# EUROPEAN PATENT APPLICATION

(11) **EP 4 456 076 A1**
(43) Date of publication of application: **30.10.2024**
(21) Application number: 22910998.8
(22) Date of filing: 13.12.2022
(51) Int. Cl.: G16C 20/30, G06F 30/27, G16C 20/70, G16C 60/00

(54) **DESIGN SUPPORT DEVICE, DESIGN SUPPORT METHOD, AND PROGRAM**

(30) Priority: 20.12.2021 JP 2021206288
(71) Applicant: Resonac Corporation, Tokyo 105-7325 (JP)
(72) Inventor: NISHINO, Shogo, Tokyo 105-8518 (JP); AONUMA, Naoto, Tokyo 105-8518 (JP); MIYAKOSHI, Koji, Tokyo 105-8518 (JP); KAKUDA, Kohsuke, Tokyo 105-8518 (JP); MINAMI, Takuya, Tokyo 105-8518 (JP); TAKEMOTO, Shimpei, Tokyo 105-8518 (JP)
(74) Representative: Mewburn Ellis LLP
(86) International application number: PCT/JP2022/045780
(87) International publication number: WO 2023/120290

(57) **Abstract**

A design support device supports design of a first compound and design of a second compound containing the first compound, and the design support device includes a first design condition proposing unit configured to propose a candidate for design condition information for the first compound that satisfies required property information for the first compound that is input; and a first property predicting unit configured to perform property prediction of the second compound based on design condition information for the second compound that is input.

## Description

### TECHNICAL FIELD

The present disclosure relates to a design support device, a design support method, and a program.

### BACKGROUND

A system that acquires composition data and property data of a photosensitive resin composition and performs a learning process on a model that outputs recommended composition data in response to target property data being input, by using training data including the acquired composition data and property data is conventionally known, for example. In a conventional system, in response to target property data being input into a model, a recommended composition for producing a photosensitive resin composition having the target property is output (see, for example, Patent Document 1).

### Related Art Document

### Patent Document

Patent Document 1: Japanese Laid-open Patent Application Publication No. 2020-77346

### SUMMARY

### Problem to be solved by the invention

It is convenient for a design support device that supports design of a resin composition (a second compound) containing a polymer (a first compound) to have a technique suitable for adjusting the property of the polymer to control the property of the resin composition, for example. Here, Patent Document 1 does not describe such contents.

An object of the present disclosure is to provide a design support device, a design support method, and a program suitable for designing a first compound and designing a second compound containing the first compound.

### Means for Solving Problem

The present disclosure includes the following configurations.
[1] A design support device that supports design of a first compound and design of a second compound containing the first compound, the design support device including:
   a first design condition proposing unit configured to propose a candidate for design condition information for the first compound that satisfies required property information for the first compound that is input; and
   a first property predicting unit configured to perform property prediction of the second compound based on design condition information for the second compound that is input.
[2] The design support device described in [1], further comprising:
   a second design condition proposing unit configured to propose a candidate for design condition information for the second compound that satisfies required property information for the second compound that is input;
   a second property predicting unit configured to perform property prediction of the first compound based on design condition information for the first compound that is input; and
   a control unit configured to support design of the first compound by using the first design condition proposing unit or the second property predicting unit, and support design of the second compound by using the second design condition proposing unit or the first property predicting unit.
[3] A design support device that supports design of a first compound and design of a second compound containing the first compound, the design support device including:
   a second property predicting unit configured to perform property prediction of the first compound based on design condition information for the first compound that is input; and
   a second design condition proposing unit configured to propose a candidate for design condition information for the second compound that satisfies required property information for the second compound that is input.
[4] The design support device described in [3], further including:
   a first design condition proposing unit configured to propose a candidate for design condition information for the first compound that satisfies required property information for the first compound that is input;
   a first property predicting unit configured to perform property prediction of the second compound based on design condition information for the second compound that is input; and
   a control unit configured to support design of the first compound by using the first design condition proposing unit or the second property predicting unit, and support design of the second compound by using the second design condition proposing unit or the first property predicting unit.
[5] The design support device described in [2] or [4], wherein the control unit is configured to perform the property prediction of the first compound based on the design condition information for the first compound by using the second property predicting unit, and perform the property prediction of the second compound based on the design condition information for the second compound containing the first compound of which the property prediction is performed, by using the first property predicting unit.
[6] The design support device described in [2] or [4], wherein the control unit is configured to propose the candidate for the design condition information for the first compound that satisfies the required property information for the first compound by using the first design condition proposing unit, and perform the property prediction of the second compound based on the design condition information for the second compound containing the first compound of the candidate for the design condition information by using the first property predicting unit.
[7] The design support device described in [2] or [4], wherein the control unit is configured to perform the property prediction of the first compound based on the design condition information for the first compound by using the second property predicting unit, and propose the candidate for the design condition information for the second compound that satisfies the required property information for the second compound containing the first compound of which the property prediction is performed, by using the second design condition proposing unit.
[8] The design support device described in [2] or [4], wherein the control unit is configured to propose the candidate for the design condition information for the first compound that satisfies the required property information for the first compound by using the first design condition proposing unit, and propose the candidate for the design condition information for the second compound that satisfies the required property information for the second compound containing the first compound of the candidate for the design condition information by using the second design condition proposing unit.
[9] The design support device described in [8], wherein the second design condition proposing unit is configured to propose a candidate for a property of the first compound that satisfies the required property information for the second compound.
[10] The design support device described in any one of [2], [4], [5], and [7], the design support device further including:
   a storage unit configured to store a result of the property prediction of the first compound performed by the second property predicting unit based on the design condition information for the first compound,
   wherein the first property predicting unit is configured to use the design condition information for the first compound and the result of the property prediction of the first compound that are read from the storage unit for input of the design condition information for the second compound and for the property prediction of the second compound.
[11] The design support device described in any one of claims [2] to [10], wherein the first design condition proposing unit and the second design condition proposing unit are configured to receive the required property information and range information on the design condition information as inputs, and propose the candidate for the design condition information for the first compound or the second compound that satisfies the required property information for the first compound or the second compound within a range of the range information that is input.
[12] The design support device described in [11], wherein the first design condition proposing unit and the second design condition proposing unit are configured to generate an exhaustive search point within the range of the range information that is input and propose the candidate for the design condition information for the first compound or the second compound that satisfies the required property information for the first compound or the second compound based on a result of the property prediction of the first compound or the second compound performed using the exhaustive search point.
[13] The design support device described in any one of [1] to [12], wherein the first compound is a polymer polymerized from monomers, and the second compound is a resin composition containing the polymer.
[14] A design support method of a computer supporting design of a first compound and design of a second compound containing the first compound, the design support method including:
   a design condition proposing step of proposing a candidate for design condition information for the first compound that satisfies required property information for the first compound that is input; and
   a property predicting step of performing property prediction of the second compound based on design condition information for the second compound that is input.
[15] A program for causing a computer configured to support design of a first compound and design of a second compound containing the first compound to execute:
   a design condition proposing procedure of proposing a candidate for design condition information for the first compound that satisfies required property information for the first compound that is input; and
   a property predicting procedure of performing property prediction of the second compound based on design condition information for the second compound
   that is input.

### Effect of the invention

According to the present disclosure, a design support device, a design support method, and a program suitable for designing a first compound and designing a second compound containing the first compound can be provided.

### BRIEF DESCRIPTION OF THE DRAWINGS

[FIG. 1] FIG. 1 is a configuration diagram of an example of an information processing system according to the present embodiment.
[FIG. 2] FIG. 2 is a hardware configuration diagram of an example of a computer according to the present embodiment.
[FIG. 3] FIG. 3 is a functional configuration diagram of an example of the information processing system according to the present embodiment.
[FIG. 4] FIG. 4 is an explanatory diagram illustrating an example of polymer synthesis and resin composition generation according to the present embodiment.
[FIG. 5A] FIG. 5A is an explanatory diagram illustrating an example of a forward problem analysis according to the present embodiment.
[FIG. 5B] FIG. 5B is an explanatory diagram illustrating an example of the forward problem analysis according to the present embodiment.
[FIG. 5C] FIG. 5C is an explanatory diagram illustrating an example of an inverse problem analysis according to the present embodiment.
[FIG. 5D] FIG. 5D is an explanatory diagram illustrating an example of the inverse problem analysis according to the present embodiment.
[FIG. 6] FIG. 6 is a flowchart illustrating an example of a processing procedure of the information processing system according to the present embodiment.
[FIG. 7] FIG. 7 is an image diagram of an example of a design mode selection screen.
[FIG. 8] FIG. 8 is a flowchart of an example of a polymer design support process using the forward problem analysis and a resin composition design support process using the forward problem analysis.
[FIG. 9A] FIG. 9A is a configuration diagram illustrating an example of polymer design condition information.
[FIG. 9B] FIG. 9B is a configuration diagram illustrating an example of polymer property prediction information.
[FIG. 10A] FIG. 10A is a configuration diagram illustrating an example of resin composition design condition information.
[FIG. 10B] FIG. 10B is a configuration diagram illustrating an example of resin composition property prediction information.
[FIG. 11] FIG. 11 is a flowchart of an example of the polymer design support process using the inverse problem analysis and the resin composition design support process using the forward problem analysis.
[FIG. 12] FIG. 12 is a configuration diagram illustrating an example of required property information for a polymer and a resin composition.
[FIG. 13] FIG. 13 is a configuration diagram illustrating an example of range information on the polymer design condition information.
[FIG. 14] FIG. 14 is a configuration diagram illustrating an example of the resin composition design condition information.
[FIG. 15A] FIG. 15A is a configuration diagram illustrating an example of information on a proposed polymer.
[FIG. 15B] FIG. 15B is a configuration diagram illustrating an example of information on the proposed polymer.
[FIG. 16] FIG. 16 is a screen image of an example of displaying the information on the proposed polymer.
[FIG. 17] FIG. 17 is a flowchart of an example of the polymer design support process using the forward problem analysis and the resin composition design support process using the inverse problem analysis.
[FIG. 18] FIG. 18 is a configuration diagram illustrating an example of the required property information for the resin composition.
[FIG. 19] FIG. 19 is a configuration diagram illustrating an example of the range information on the resin composition design condition information.
[FIG. 20A] FIG. 20A is a configuration diagram illustrating an example of information on a proposed resin composition.
[FIG. 20B] FIG. 20B is a configuration diagram illustrating an example of the information on the proposed resin composition.
[FIG. 21] FIG. 21 is a flowchart of an example of the polymer design support process using the inverse problem analysis and the resin composition design support process using the inverse problem analysis.
[FIG. 22A] FIG. 22A is a configuration diagram illustrating an example of the information on the proposed polymer.
[FIG. 22B] FIG. 22B is a configuration diagram illustrating an example of the information on the proposed polymer.
[FIG. 23] FIG. 23 is a configuration diagram illustrating an example of the range information on the resin composition design condition information.
[FIG. 24A] FIG. 24A is a configuration diagram illustrating an example of the information on the proposed resin composition.
[FIG. 24B] FIG. 24B is a configuration diagram illustrating an example of the information on the proposed resin composition.
[FIG. 25] FIG. 25 is a flowchart of an example of the polymer design support process using the inverse problem analysis and the resin composition design support process using the inverse problem analysis.
[FIG. 26] FIG. 26 is a configuration diagram illustrating an example of the range information on the polymer property prediction information.
[FIG. 27A] FIG. 27A is a configuration diagram illustrating an example of the information on the proposed polymer.
[FIG. 27B] FIG. 27B is a configuration diagram illustrating an example of the information on the proposed polymer.
[FIG. 28] FIG. 28 is a configuration diagram illustrating an example of required property information for a polymer that is transcribed from the information on the proposed polymer.
[FIG. 29A] FIG. 29A is a configuration diagram illustrating an example of the information on the proposed polymer.
[FIG. 29B] FIG. 29B is a configuration diagram illustrating an example of the information on the proposed polymer.
[FIG. 30] FIG. 30 is a flowchart of an example of a process of registering and using the design condition information and the property prediction information.
[FIG. 31A] FIG. 31A is a configuration diagram illustrating an example of the polymer design condition information.
[FIG. 31B] FIG. 31B is a configuration diagram illustrating an example of the polymer property prediction information.
[FIG. 32A] FIG. 32A is a configuration diagram illustrating an example of a process of registering and using the design condition information and the property prediction information on the polymer.
[FIG. 32B] FIG. 32B is a configuration diagram illustrating an example of a process of registering and using the design condition information and the property prediction information on the polymer.
[FIG. 33] FIG. 33 is a configuration diagram illustrating an example of the resin composition property prediction information.

### DESCRIPTION OF EMBODIMENTS

Next, embodiments of the present invention will be described in detail. Here, the present invention is not limited to the following embodiments. In the present embodiment, a polymer and a resin composition containing the polymer will be described as an example of a first compound and a second compound containing the first compound.

### [First Embodiment]

### <System Configuration>

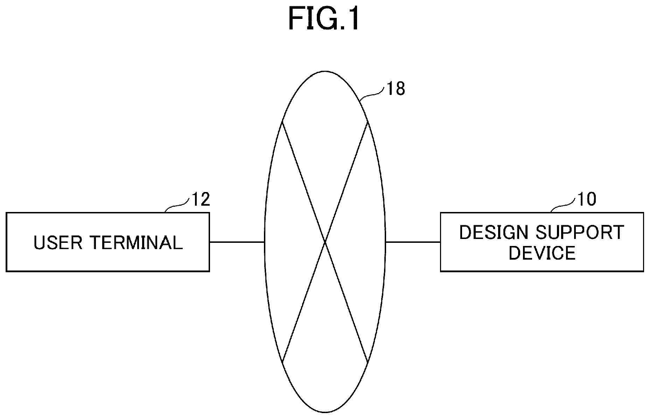

FIG. 1 is a configuration diagram of an example of an information processing system according to the present embodiment. The information processing system of FIG. 1 includes a design support device 10 and a user terminal 12. The design support device 10 and the user terminal 12 are connected to each other via a communication network 18 such as a local area network (LAN) or the Internet so that data communication can be performed.

The user terminal 12 is an information processing terminal operated by an operator, such as a PC, a tablet terminal, or a smartphone. The user terminal 12 displays a screen for receiving an input of information from the operator on a display device and receives an input of the information from the operator. Additionally, the user terminal 12 transmits the information received from the operator to the design support device 10, and causes the design support device 10 to perform processing related to the design of the polymer and the resin composition and the prediction of the property. The user terminal 12 receives information on a result of the processing performed by the design support device 10, displays the information on the display device to allow the operator to confirm the information.

The design support device 10 is an information processing device, such as a PC, that supports design of a polymer and a resin composition. The design support device 10 performs processing of an inverse problem analysis for proposing a candidate for a design condition that satisfies required properties and processing of a forward problem analysis for performing property prediction based on the design condition. The design support device 10 supports the design of the polymer and the resin composition by combining the design of the polymer and the resin composition by the inverse problem analysis (search and optimization) and the property prediction of the polymer and the resin composition by the forward problem analysis.

As the combinations of the design supports, specifically, a combination of a polymer design by the inverse problem analysis and a property prediction of a resin composition by the forward problem analysis, a combination of a polymer design by the forward problem analysis and a resin composition design by the inverse problem analysis, a combination of a polymer design by the forward problem analysis and a property prediction of a resin composition by the forward problem analysis, or a combination of a polymer design by the inverse problem analysis and a resin composition design by the inverse problem analysis can be used.

For example, the design support device 10 is effective when searching and optimizing (the inverse problem analysis) a design condition of a polymer when synthesizing the polymer from monomers, and then evaluating (the forward problem analysis) a property of a resin composition in which a polymerization initiator, a sensitizer, an adhesion agent, a plasticizer, and the like are mixed with the synthesized polymer.

In the forward problem analysis that performs the property prediction based on the design condition, a mathematical model, such as a machine learning model that has learned a correspondence relationship between the design condition and the property, can be used. As the machine learning method, for example, a supervised learning method such as linear, generalized linear (Lasso, ridge, elastic net, logistic), partial least squares, kernel ridge, a Gaussian process, a k-nearest neighbor method, a decision tree, random forest, AdaBoost, bagging, gradient boosting, a support vector machine, or a neural network may be used.

In the inverse problem analysis for proposing a candidate for a design condition that satisfies required properties, the required properties and a range of the design condition are input, and exhaustive search points are generated at random or with predetermined step sizes in the range of the design condition. Additionally, in the inverse problem analysis for proposing a candidate for the design condition that satisfies the required properties, properties corresponding to each exhaustive search point are predicted by a mathematical model, and an exhaustive search point having properties that satisfy the required properties is extracted to obtain the candidate for the design condition that satisfies the desired required properties.

The inverse problem analysis for proposing the candidate for the design condition that satisfies the required properties may use an optimization method, such as a genetic algorithm, a simulated annealing method, a cluster algorithm, or an extended ensemble method (for example, a multi-canonical method, a simulated tempering method, a replica exchange Monte Carlo method (parallel tempering method), or the like).

When the property prediction of the polymer and the resin composition is performed by the mathematical model, the mixing amount of each material may be input as an explanatory variable, or information that does not directly indicate a physicochemical property, for example, the brands of the polymer and the resin composition may be described by a dummy variable represented by "0" and "1", and may be used as an explanatory variable. Additionally, when the property prediction of the polymer and the resin composition is performed by the mathematical model, explanatory variables may be created and input based on information obtained by quantifying a structural characteristic of a molecule being a material or information obtained by quantifying a chemical characteristic of the molecule, and the mixing amount thereof.

As an example of a method of quantifying a structural characteristic of a molecule, Extended Circular Fingerprints (hereinafter referred to as ECFP) is used. The ECFP is a method of extracting the types and numbers of all partial structures and expressing them as vectors (column: the type, value: the number) to quantify a structural characteristic of a molecule.

The partial structure can be expressed by a notation method such as Simplified Molecular Input Line Entry System (hereinafter referred to as SMILES) notation. The ECFP can calculate the structural characteristic of the molecule, for example, by inputting information on an additive represented by the SMILES notation into an existing library. Additionally, another example of a method of quantifying the structural characteristic of the molecule is a graph convolution neural network. The graph convolutional neural network can calculate structural characteristic of the molecule, for example, by inputting information on an additive represented by the SMILES notation into an existing library.

Additionally, for example, as an example of a method of quantifying a chemical characteristic of a molecule, a method of extracting physical property information of the molecule, and representing it as a vector (column: the physical property information, value: a numerical number) to quantify the chemical characteristic of the molecule is used. A method of quantifying the chemical characteristic of the molecule can calculate the chemical characteristic of the molecule, for example, by inputting information on an additive represented by the SMILES notation into an existing library.

The physical property information of a molecule is, for example, the molecular weight, the number of valence electrons, the partial charge, the number of amino groups, the number of hydroxyl groups, and the like. Additionally, the physical property information of a molecule may be a physical property value that can be calculated by quantum chemical calculation software, such as HOMO, LUMO, the electric charge, the refractive index, or the frequency, or a physical property value that can be measured by an experiment, such as melting point, viscosity, or specific surface area.

The design support device 10 receives the information input to the user terminal 12 by the operator, and performs the processing related to the design and the property prediction of a polymer and a resin composition. The design support device 10 transmits the information on a result of the processing to the user terminal 12, displays the result on the user terminal 12, and allows the operator to confirm the result. The information processing system illustrated in FIG. 1 may be realized by, for example, the design support device 10 having a web server function and the user terminal 12 that executes a web application by using a web browser function.

Here, the information processing system of FIG. 1 is an example, and it is needless to say that there are various system configuration examples according to applications and purposes. For example, the design support device 10 may be realized by multiple computers, or may be realized as a cloud computing service. Additionally, the information processing system of FIG. 1 may be realized by a stand-alone computer.

### <Hardware Configuration>

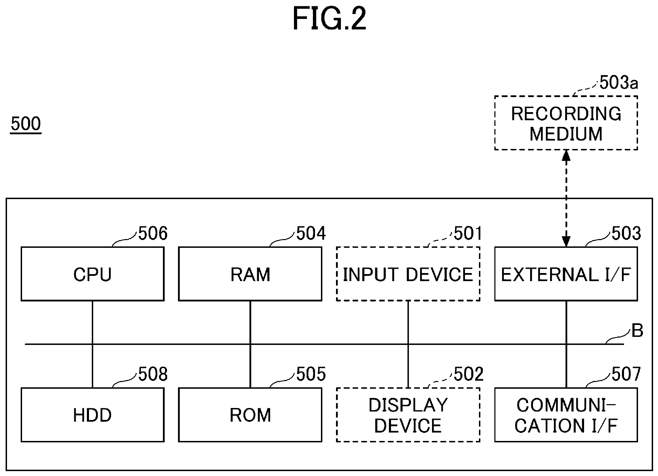

The design support device 10 and the user terminal 12 of FIG. 1 are realized by, for example, a computer 500 having a hardware configuration illustrated in FIG. 2.

FIG. 2 is a hardware configuration diagram of an example of a computer according to the present embodiment. The computer 500 of FIG. 2 includes an input device 501, a display device 502, an external I/F 503, a RAM 504, a ROM 505, a CPU 506, a communication I/F 507, an HDD 508, and the like, which are connected to each other via a bus B. Here, the input device 501 and the display device 502 may be configured to be used by being connected.

The input device 501 is a touch panel, an operation key and a button, a keyboard and a mouse, or the like used by a user to input various signals. The display device 502 includes a display such as a liquid crystal display or an organic EL display that displays a screen, a speaker that outputs sound data such as voice and sound, and the like. The communication I/F 507 is an interface for the computer 500 to perform data communication.

Additionally, the HDD 508 is an example of a non-volatile storage device that stores programs and data. The stored programs and data include an OS, which is basic software that controls the entirety of the computer 500, applications that provide various functions on the OS, and the like. Here, the computer 500 may use a drive device (for example, a solid state drive (SSD)) using a flash memory as a storage media instead of the HDD 508.

The external I/F 503 is an interface with an external device. The external device includes a recording medium 503a and the like. This allows the computer 500 to read from and/or write to the recording medium 503a via the external I/F 503. The recording medium 503a includes a flexible disk, a CD, a DVD, an SD memory card, a USB memory, and the like.

The ROM 505 is an example of a non-volatile semiconductor memory (a storage device) that can retain programs and data when the power is turned off. The ROM 505 stores programs and data such as a BIOS, which is executed when the computer 500 is activated, OS settings, network settings, and the like. The RAM 504 is an example of a volatile semiconductor memory (a storage device) that temporarily stores programs and data.

The CPU 506 is an arithmetic device that reads the program and data from the storage device such as the ROM 505 or the HDD 508 onto the RAM 504 and executes processing to control the entirety of the computer 500 and realize functions thereof. The computer 500 according to the present embodiment can realize various functions of the design support device 10 and the user terminal 12, as will be described later, by executing the program.

### <Functional Configuration>

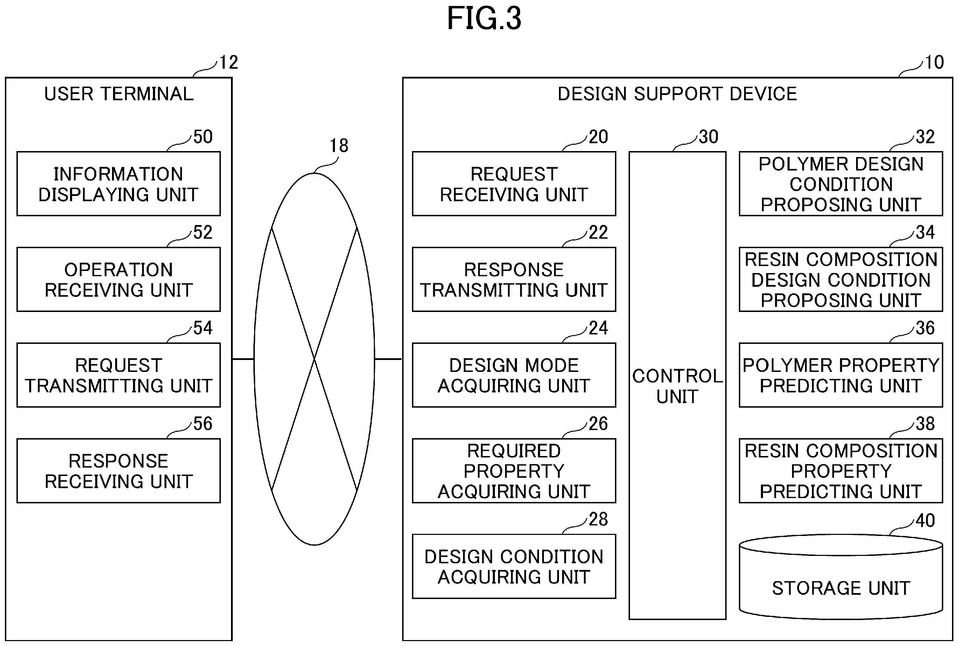

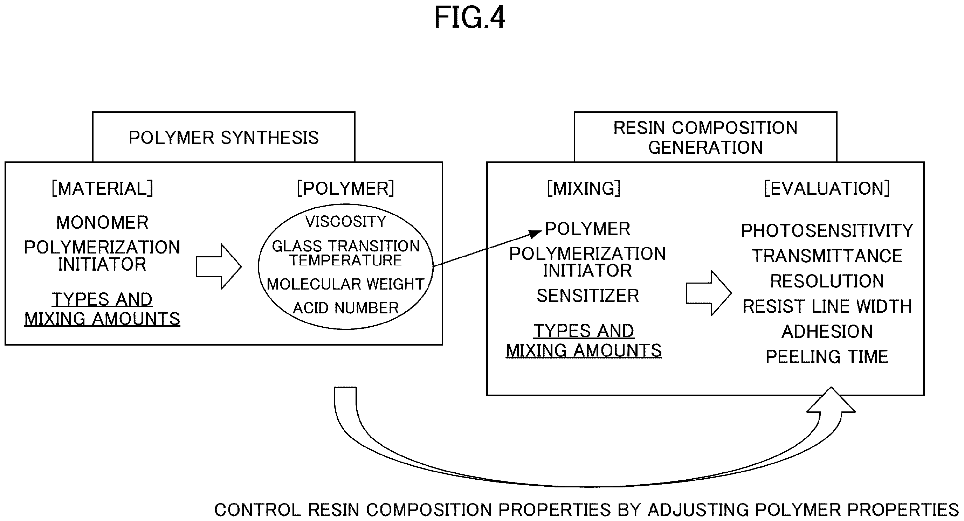

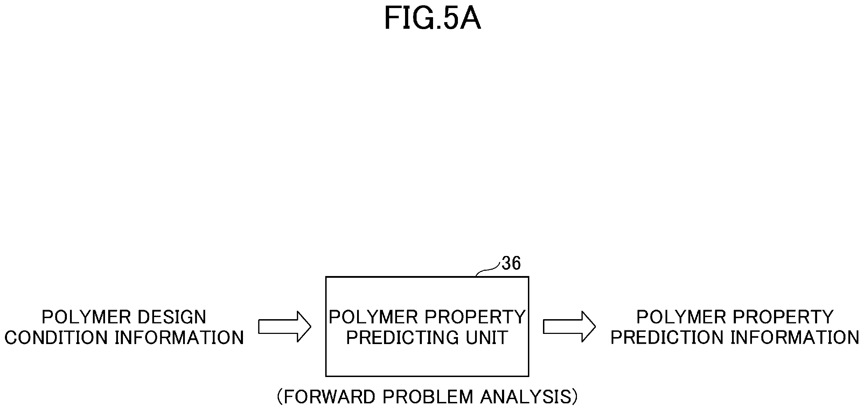

A functional configuration of the information processing system according to the present embodiment will be described. FIG. 3 is a functional configuration diagram of an example of the information processing system according to the present embodiment. Here, in the configuration diagram of FIG. 3, portions unnecessary for the description of the present embodiment are omitted as appropriate. FIG. 4 is an explanatory diagram illustrating an example of polymer synthesis and resin composition generation according to the present embodiment. Additionally, FIGS. 5A to 5D are explanatory diagrams illustrating examples of the forward problem analysis and the inverse problem analysis according to the present embodiment.

The information processing system according to the present embodiment supports, for example, polymer synthesis and resin composition generation illustrated in FIG. 4. A material of a polymer is, for example, a monomer, a polymerization initiator, a polymerization inhibitor, a solvent used for polymerization, and the like. The design conditions of the polymer include types and mixing amounts of materials, the temperature for treatment such as heat treatment, the treatment time, and the like. Properties of the polymer include the viscosity, the glass transition temperature, the molecular weight, the acid number, and the like. The polymer is synthesized, for example, as illustrated in FIG. 4.

The resin composition is generated, for example, as illustrated in FIG. 4, by mixing a synthesized polymer with a polymerization initiator, a sensitizer, an adhesion agent, a plasticizer, and the like. The resin composition may be a liquid, gel, or paste resin. Additionally, the resin composition may be a film, a film laminate, a composite material of a film and a metal, or a processed resin product such as a molded product. Additionally, the resin composition may be for metal wiring.

Examples of the material of the resin composition include a polymer, an oligomer, a monomer, a filler, a catalyst, a polymerization initiator, a polymerization inhibitor, a crosslinking agent, a curing agent, a plasticizer, a thickener, a solvent, and the like. Additionally, the material of the resin composition may be an alkali-soluble polymer, an ethylenically unsaturated bond-containing compound, a photopolymerization initiator, a resin having a repeating unit containing an acid-decomposable group, a phenol resin, a photoacid generator, a dissolution inhibitor, a sensitizer, an adhesion agent, or the like. The design conditions of the resin composition include the types and amounts of the materials, the temperature for treatment such as heat treatment, the treatment time, the coating temperature when coating in the form of a film, the coating speed, and the like.

Additionally, the properties of the resin composition include the film thickness, the photosensitivity, the transmittance, the resolution, the developability (the minimum development time, the development rate, the development form, the development residue), the minimum resist line width, the adhesion to a substrate, the developer foaming property, the developer cohesion property, the edge fuse property, the cured film flexibility, the tackiness to a base film, the tackiness to a cover film, the hue stability, the color difference, peeling time, the peeled piece size, the tenting property, the dispersibility, the solvent resistance (e.g., N-methyl-2-pyrrolidone (NMP) resistance), the heat resistance (e.g., a yellowing color difference), and the like.

The properties of the resin composition can be controlled by adjusting the properties of the polymer, such as the viscosity, the glass transition temperature, the molecular weight, and the acid value of the polymer, for example, as illustrated in FIG. 4. Additionally, when the property prediction of the resin composition is performed by a mathematical model, one or both of polymer design condition information used when the polymer is synthesized from monomers and polymer property prediction information may be used in addition to the mixing amount of the synthesized polymer and the types and mixing amounts of the polymerization initiator, the sensitizer, the adhesive agent, the plasticizer, and the like to be mixed.

Additionally, for example, as illustrated in FIG. 4, in the information processing system according to the present embodiment, even if materials having the same name are used in the polymer synthesis stage and the resin composition preparation stage, the chemical actions and roles of the materials having the same name in the polymer synthesis stage and the resin composition preparation stage can be appropriately distinguished.

The design support device 10 of the information processing system illustrated in FIG. 3 includes a request receiving unit 20, a response transmitting unit 22, a design mode acquiring unit 24, a required property acquiring unit 26, a design condition acquiring unit 28, a control unit 30, a polymer design condition proposing unit 32, a resin composition design condition proposing unit 34, a polymer property predicting unit 36, a resin composition property predicting unit 38, and a storage unit 40. Additionally, the user terminal 12 includes an information displaying unit 50, an operation receiving unit 52, a request transmitting unit 54, and a response receiving unit 56.

The information displaying unit 50 displays, on the display device 502, a screen for receiving input of information from an operator and information such as a result of processing performed by the design support device 10. The operation receiving unit 52 receives an operation of the operator such as an input of information. The request transmitting unit 54 transmits, to the design support device 10, a request for processing corresponding to the input of the information from the operator. Additionally, the response receiving unit 56 receives, from the design support device 10, a response to the request for the processing transmitted by the request transmitting unit 54.

The request receiving unit 20 receives the request for the processing from the user terminal 12. The response transmitting unit 22 responds with a processing result corresponding to the request for the processing. The design mode acquiring unit 24 acquires information on a design mode, which will be described later, selected by the operator on the user terminal 12. The required property acquiring unit 26 acquires required property information for the polymer and the resin composition. The design condition acquiring unit 28 acquires design condition information for the polymer and the resin composition. Additionally, the design condition acquiring unit 28 acquires range information on the design condition information for the polymer and the resin composition.

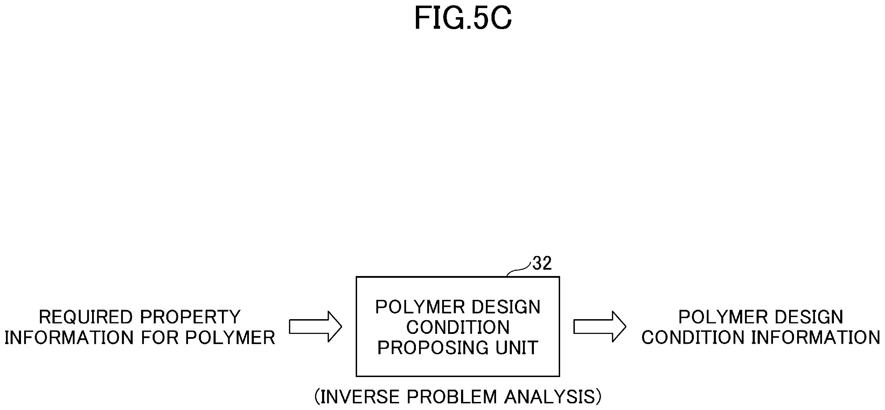

The polymer design condition proposing unit 32 is an example of a first design condition proposing unit. As illustrated in FIG. 5C, the polymer design condition proposing unit 32 proposes, by using a mathematical model, a candidate for the polymer design condition information that satisfies the input required property information for the polymer.

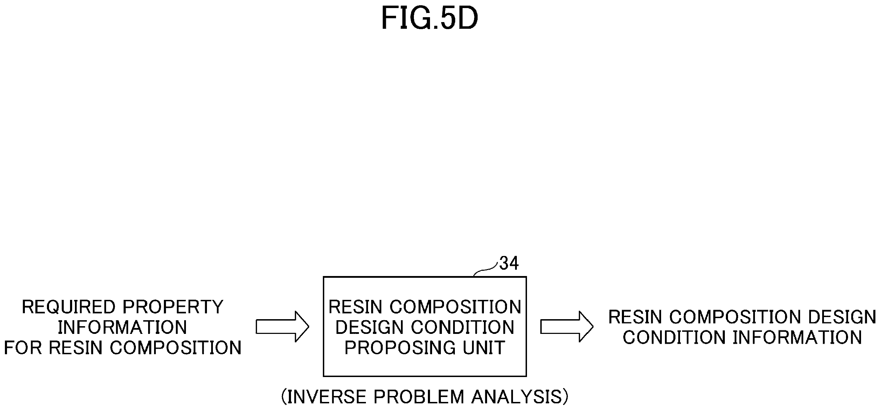

The resin composition design condition proposing unit 34 is an example of a second design condition proposing unit. As illustrated in FIG. 5D, the resin composition design condition proposing unit 34 proposes, by using a mathematical model, a candidate for the resin composition design condition information that satisfies the input required property information for the resin composition.

The polymer property predicting unit 36 is an example of a second property predicting unit. As illustrated in FIG. 5A, the polymer property predicting unit 36 performs property prediction of the polymer based on the input polymer design condition information by using a mathematical model, and outputs polymer property prediction information.

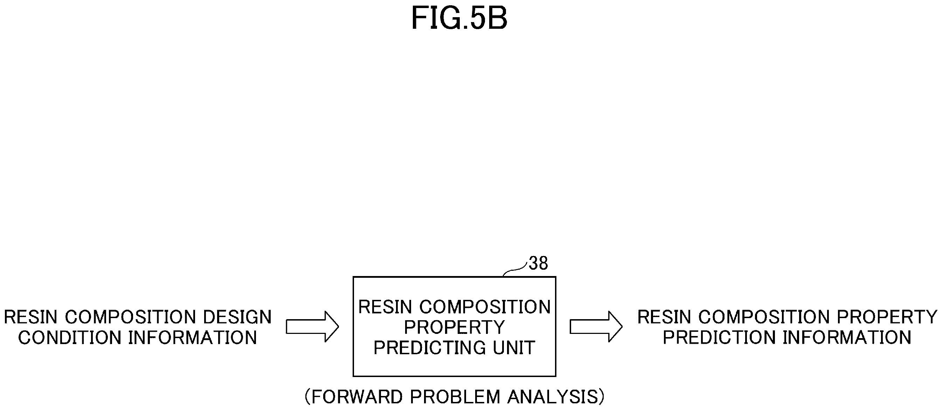

The resin composition property predicting unit 38 is an example of a first property predicting unit. As illustrated in FIG. 5B, the resin composition property predicting unit 38 performs property prediction of the resin composition based on the input resin composition design condition information by using a mathematical model, and outputs resin composition property prediction information. The resin composition design condition information includes the polymer design condition information input to the polymer property predicting unit 36 and the polymer property prediction information output from the polymer property predicting unit 36.

Based on the information on the design mode selected by the operator, the control unit 30 supports the design of the polymer by using the polymer design condition proposing unit 32 or the polymer property predicting unit 36, and supports the design of the resin composition by using the resin composition design condition proposing unit 34 or the resin composition property predicting unit 38. Additionally, the storage unit 40 stores mathematical models used by the polymer design condition proposing unit 32, the resin composition design condition proposing unit 34, the polymer property predicting unit 36, and the resin composition property predicting unit 38. The storage unit 40 stores the polymer design condition information input to the polymer property predicting unit 36 and the polymer property prediction information output from the polymer property predicting unit 36 in association with each other. The storage unit 40 stores the resin composition design condition information input to the resin composition property predicting unit 38 and the resin composition property prediction information output from the resin composition property predicting unit 38 in association with each other. The storage unit 40 may store the required property information for the polymer input to the polymer design condition proposing unit 32 and the candidate for the polymer design condition information output from the polymer design condition proposing unit 32 in association with each other. Additionally, the storage unit 40 may store the required property information for the resin composition input to the resin composition design condition proposing unit 34 and the candidate for the resin composition design condition information output from the resin composition design condition proposing unit 34 in association with each other. Here, the configuration diagram of FIG. 3 is an example. The configuration of the information processing system according to the present embodiment can be realized by various configurations.

### <Processing>

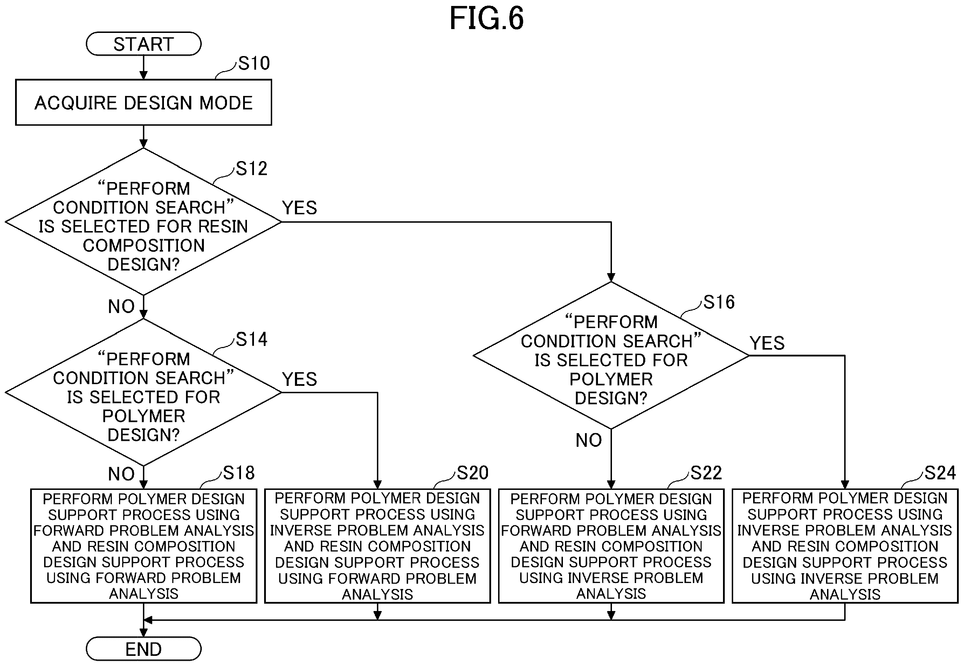

The information processing system according to the present embodiment changes the process of supporting the design of the polymer and the design of the resin composition containing the polymer as illustrated in the flowchart of FIG. 6 based on the design mode selected by the operator at the user terminal 12.

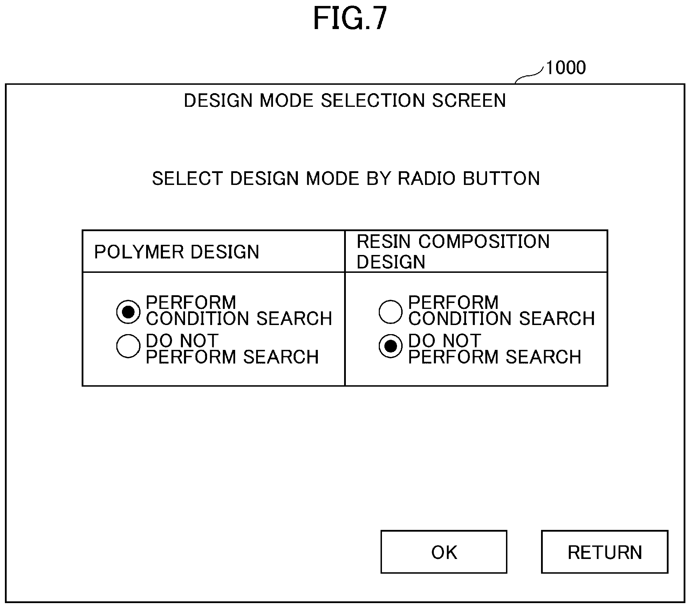

FIG. 6 is a flowchart illustrating an example of a processing procedure of the information processing system according to the present embodiment. FIG. 7 is an image diagram of an example of a design mode selection screen. In step S10, the design mode acquiring unit 24 of the design support device 10 acquires information on the design mode selected by the operator at the user terminal 12.

For example, the operator selects a design mode from a design mode selection screen 1000 of FIG. 7 displayed at the user terminal 12. The design mode selection screen 1000 of FIG. 7 is an example in which the operator is caused to select "perform condition search" or "do not perform search" of the polymer design and is caused to select "perform condition search" or "do not perform search" of the resin composition design.

The operator can select the support of the polymer design by the inverse problem analysis by selecting "perform condition search" of the polymer design on the design mode selection screen 1000. The operator can select the support of the polymer design by the forward problem analysis by selecting "do not perform search" of the polymer design on the design mode selection screen 1000.

The operator can select the support of the resin composition design by the inverse problem analysis by selecting "perform condition search" of the resin composition design on the design mode selection screen 1000. The operator can select the support of the resin composition design by the forward problem analysis by selecting "do not perform search" of the resin composition design on the design mode selection screen 1000.

In step S12, the control unit 30 of the design support device 10 determines whether the operator has selected "perform condition search" of the resin composition design mode based on the information on the design mode acquired in step S10. If the operator does not select "perform condition search" of the resin composition design mode, the control unit 30 determines that the operator has selected "do not perform search", and proceeds to the processing of step S14.

In step S14, the control unit 30 of the design support device 10 determines whether the operator has selected "perform condition search" of the polymer design based on the information on the design mode acquired in step S10. If the operator has not selected "perform condition search" of the polymer design, the control unit 30 determines that the operator has selected "do not perform search" of the polymer design, and proceeds to the processing of step S18. If the operator selects "perform condition search" of the polymer design, the control unit 30 proceeds to the processing of step S20.

In step S18, the control unit 30 performs a polymer design support process using the forward problem analysis and a resin composition design support process using the forward problem analysis. In step S20, the control unit 30 performs a polymer design support process using the inverse problem analysis and the resin composition design support process using the forward problem analysis.

Additionally, in step S12, the control unit 30 of the design support device 10 determines whether the operator has selected "perform condition search" of the resin composition design based on the information on the design mode acquired in step S10. If the operator selects "perform condition search" of the resin composition design, the control unit 30 proceeds to the processing of step S16.

In step S16, the control unit 30 of the design support device 10 determines whether the operator has selected "perform condition search" of the polymer design based on the information on the design mode acquired in step S10. If the operator has not selected "perform condition search" of the polymer design, the control unit 30 determines that the operator has selected "do not perform search" of the polymer design, and proceeds to the processing of step S22. If the operator has selected "perform condition search" of the polymer design, the control unit 30 proceeds to the processing of step S24.

In step S22, the control unit 30 performs the polymer design support process using the forward problem analysis and the resin composition design support process using the inverse problem analysis. In step S24, the control unit 30 performs the polymer design support process using the inverse problem analysis and the resin composition design support process using the inverse problem analysis.

Here, the design mode selection screen 1000 of FIG. 7 is an example, and "perform condition search" or "do not perform search" may be selected for any one of the polymer design and the resin composition design. Additionally, when the number of design modes available to the operator is one, the process illustrated in FIG. 6 may be omitted.

### <<Processing Example of Step S18>>

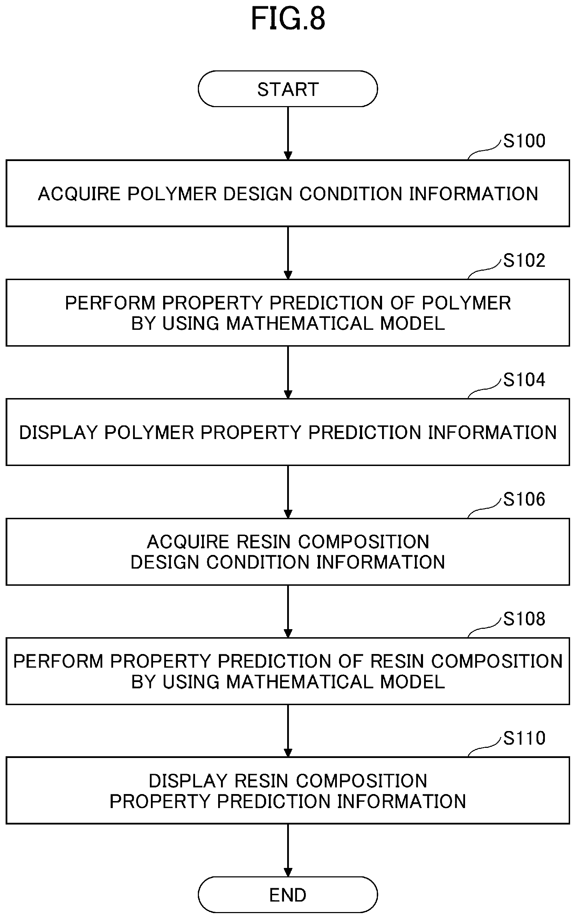

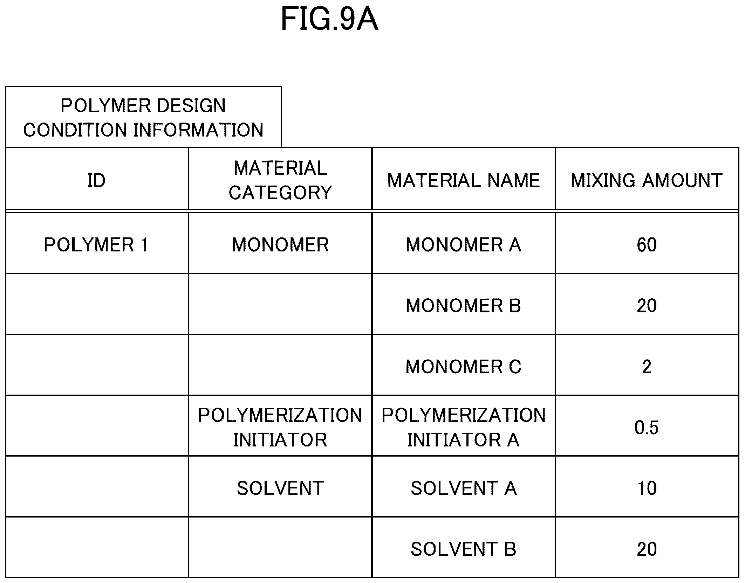

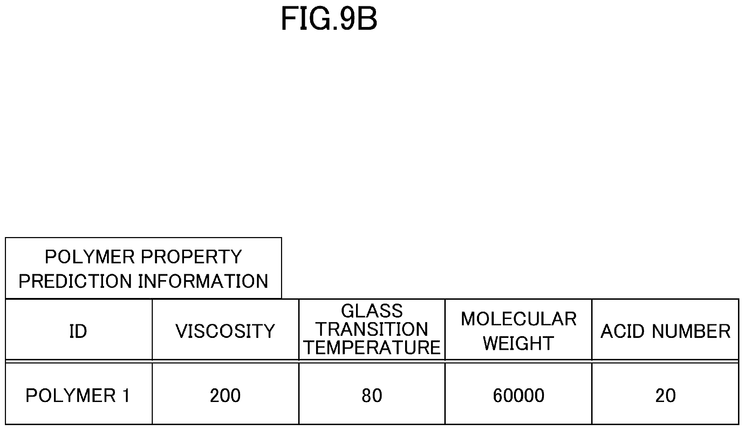

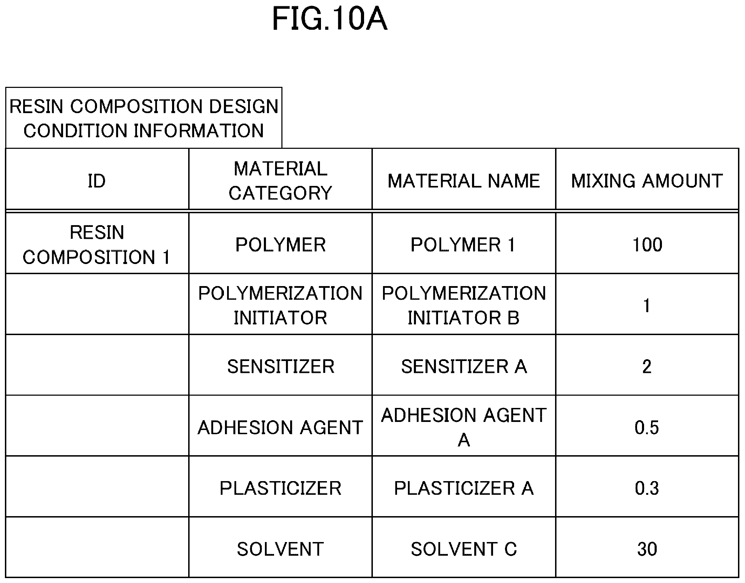

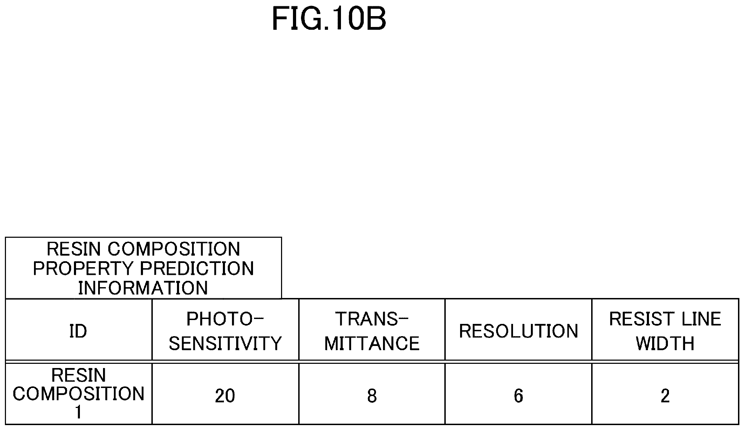

FIG. 8 is a flowchart of an example of the polymer design support process using the forward problem analysis and the resin composition design support process using the forward problem analysis. FIG. 9A is a configuration diagram illustrating an example of the polymer design condition information. FIG. 9B is a configuration diagram illustrating an example of the polymer property prediction information. FIG. 10A is a configuration diagram illustrating an example of the resin composition design condition information. FIG. 10B is a configuration diagram illustrating an example of the resin composition property prediction information.

In step S100, the design condition acquiring unit 28 of the design support device 10 acquires the polymer design condition information input by the operator at the user terminal 12, for example, as illustrated in FIG. 9A. The polymer design condition information includes the types and mixing amounts of the materials as information.

In step S102, the control unit 30 provides, to the polymer property predicting unit 36, the polymer design condition information acquired by the design condition acquiring unit 28 in step S100, and requests property prediction of the polymer. The polymer property predicting unit 36 performs the property prediction based on the provided polymer design condition information by using the mathematical model. The polymer property predicting unit 36 obtains the polymer property prediction information as illustrated in FIG. 9B, for example, by the property prediction using the mathematical model.

In step S104, the control unit 30 performs control to cause the user terminal 12 to display the polymer property prediction information acquired in step S102, for example, as illustrated in FIG. 9B. The information that the user terminal 12 is caused to display in step S104 may include the polymer design condition information that is input by the operator in step S100, for example, illustrated in FIG. 9A.

In step S106, the design condition acquiring unit 28 of the design support device 10 acquires the resin composition design condition information input by the operator at the user terminal 12, for example, illustrated in FIG. 10A. The resin composition design condition information illustrated in FIG. 10A includes the polymer whose property prediction information has been obtained in step S102 as a material. The resin composition design condition information includes types and mixing amounts of the materials as information.

In step S108, the control unit 30 provides, to the resin composition property predicting unit 38, the resin composition design condition information acquired by the design condition acquiring unit 28 in step S106, and requests the property prediction of the resin composition. The resin composition property predicting unit 38 performs the property prediction based on the provided resin composition design condition information by using the mathematical model. The resin composition property predicting unit 38 acquires the resin composition property prediction information, for example, as illustrated in FIG. 10B by the property prediction using the mathematical model.

Additionally, in step S110, the control unit 30 performs control to cause the user terminal 12 to display the resin composition property prediction information acquired in step S108, for example, as illustrated in FIG. 10B. The information that the user terminal 12 is caused to display in step S110 may include, for example, the resin composition design condition information input by the operator in step S106, for example, illustrated in FIG. 10A.

According to the process of FIG. 8, the property of the polymer when the polymer is synthesized from the monomers is evaluated, and then the property of the resin composition in which the polymerization initiator, the sensitizer, the adhesion agent, the plasticizer, the solvent, and the like are mixed with the synthesized polymer can be evaluated.

### <<Processing Example of Step S20>>

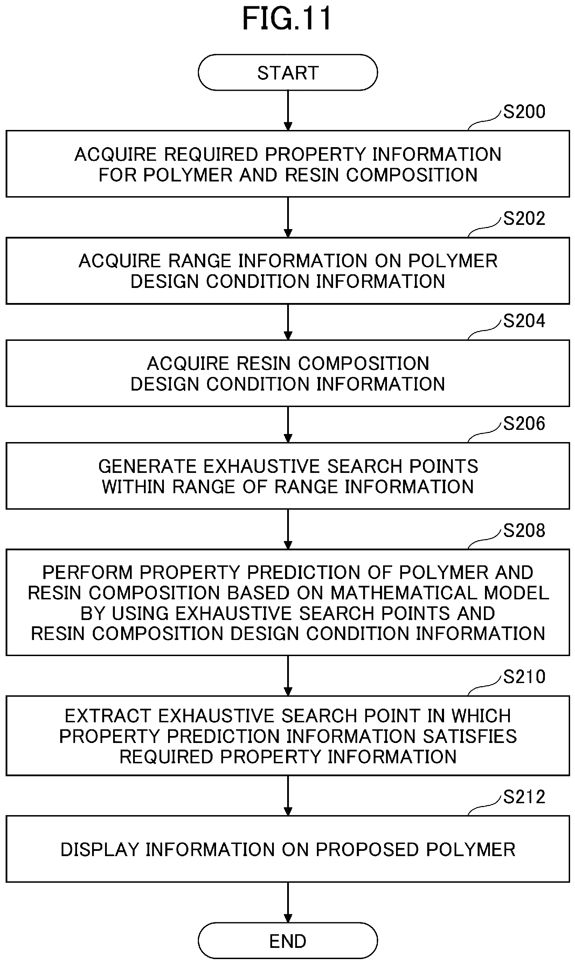

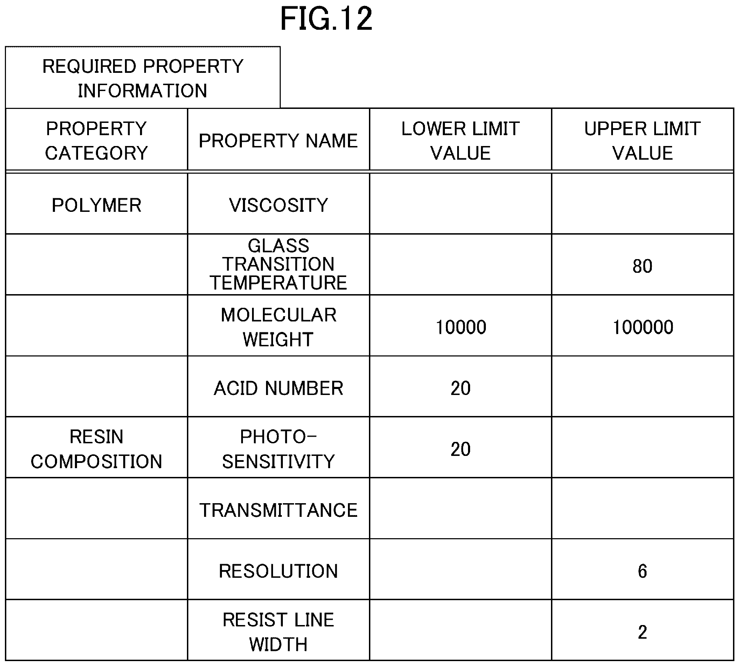

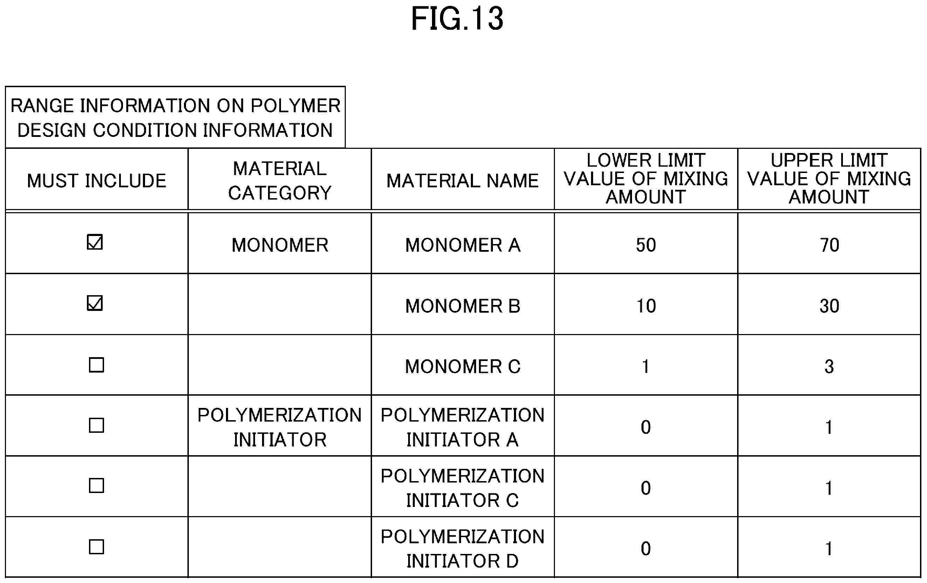

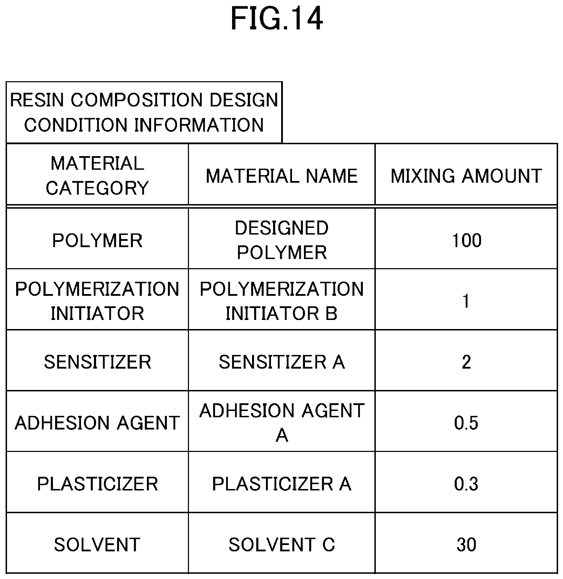

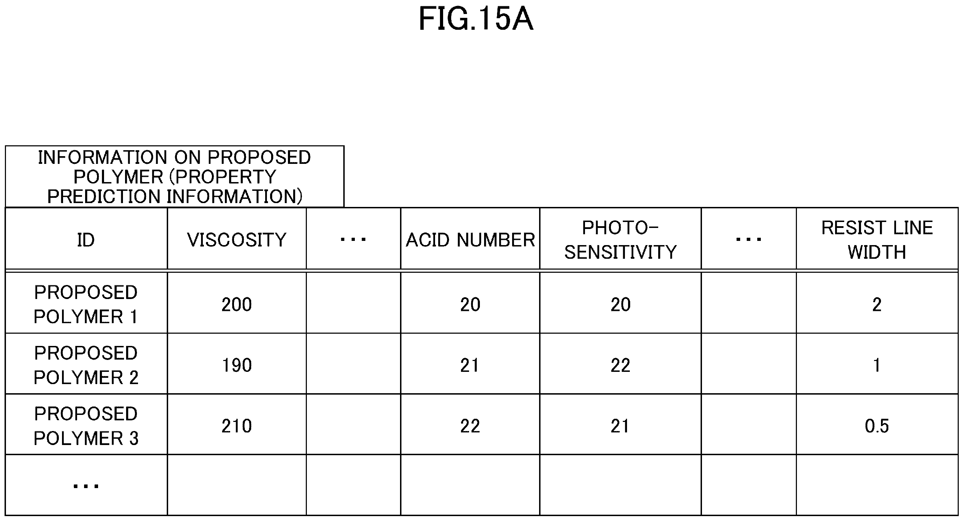

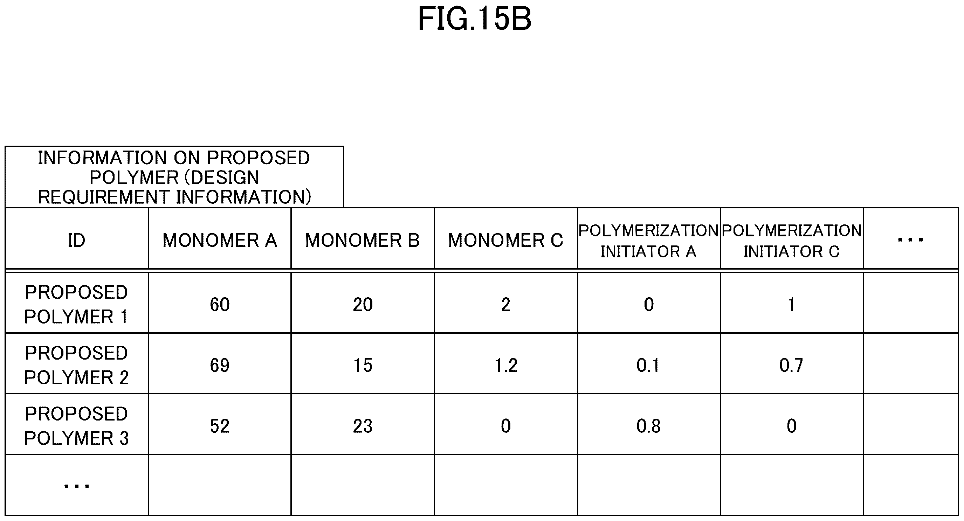

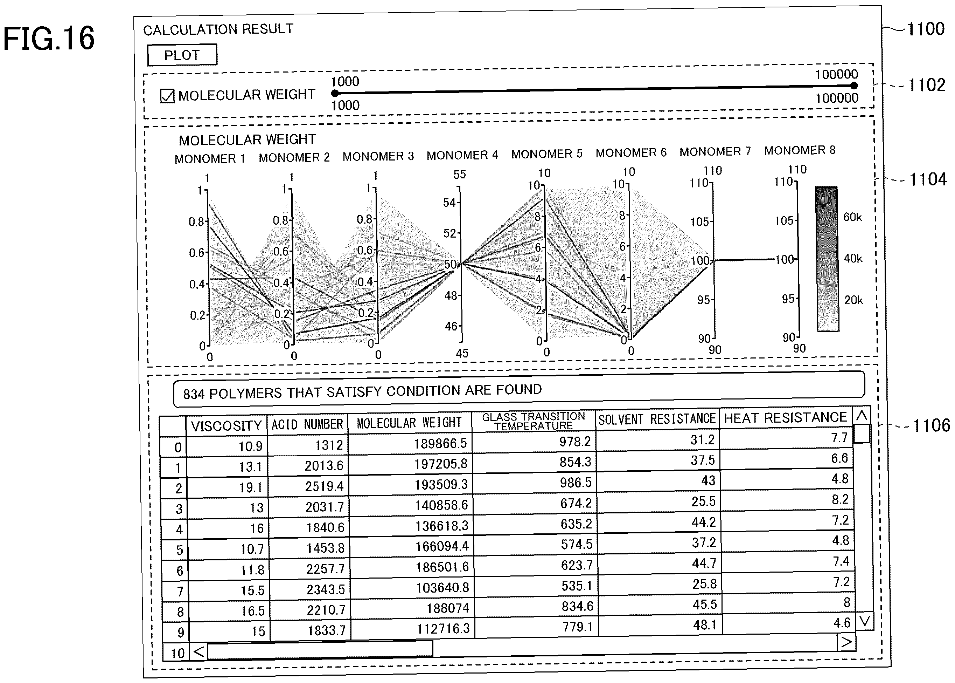

FIG. 11 is a flowchart of an example of the polymer design support process using the inverse problem analysis and the resin composition design support process using the forward problem analysis. FIG. 12 is a configuration diagram illustrating an example of the required property information for the polymer and the resin composition. FIG. 13 is a configuration diagram illustrating an example of the range information on the polymer design condition information. FIG. 14 is a configuration diagram illustrating an example of the resin composition design condition information. FIG. 15A and FIG. 15B are configuration diagrams illustrating an example of information on a proposed polymer. FIG. 16 is a screen image illustrating an example of displaying the information on the proposed polymer.

In step S200, the required property acquiring unit 26 of the design support device 10 acquires the required property information for the polymer and the resin composition input by the operator at the user terminal 12, for example, as illustrated in FIG. 12. The required property information for the polymer and the resin composition in FIG. 12 is an example in which the required properties for the polymer and the resin composition are represented by the lower limit value and the upper limit value.

In step S202, the design condition acquiring unit 28 acquires the range information on the polymer design condition information input by the operator at the user terminal 12, for example, as illustrated in FIG. 13. The range information on the polymer design condition information in FIG. 13 includes, as information, the type of material and the lower limit value and the upper limit value of the mixing amount of each material.

Additionally, the range information on the polymer design condition information in FIG. 13 is an example in which the operator can select a material that must be included in the polymer to be synthesized. The item "must include" is checked for the material selected by the operator to be necessarily included in the polymer to be synthesized. FIG. 13 indicates an example in which the operator selects the "monomer A" and the "monomer B" as the materials necessary to be included in the polymer to be synthesized.

In step S204, the design condition acquiring unit 28 acquires the resin composition design condition information input by the operator at the user terminal 12, for example, as illustrated in FIG. 14. The resin composition design condition information illustrated in FIG. 14 includes a designed polymer as a material. The resin composition design condition information includes the types and mixing amounts of the materials as information.

In step S206, the control unit 30 provides, to the polymer design condition proposing unit 32, the range information on the polymer design condition information acquired in step S202, and requests generation of the exhaustive search points within the range of the range information on the polymer design condition information. The polymer design condition proposing unit 32 generates a predetermined number (for example, 1000) of exhaustive search points at random or at predetermined intervals within the range of the range information on the provided polymer design condition information.

The exhaustive search point is a combination of mixing amounts of "monomer A", "monomer B", "monomer C", "polymerization initiator A", "polymerization initiator C", and "polymerization initiator D", which are the materials, for the range information on the polymer design condition information of FIG. 13, for example. The mixing amount of each material is selected from the range indicated by the items "lower limit value of mixing amount" and "upper limit value of mixing amount".

In step S208, the polymer design condition proposing unit 32 performs the property prediction of the polymer based on multiple exhaustive search points generated in step S206 by using the mathematical model, and obtains multiple polymer property prediction information based on the multiple exhaustive search points. The control unit 30 provides, to the resin composition property predicting unit 38, the multiple polymer property prediction information based on the multiple exhaustive search points and the resin composition design condition information acquired by the design condition acquiring unit 28 in step S204, and requests the property prediction of the resin composition.

The resin composition property predicting unit 38 performs the property prediction based on the multiple polymer property prediction information based on the provided multiple exhaustive search points and the resin composition design condition information acquired by the design condition acquiring unit 28 in step S204 by using the mathematical model. The resin composition property predicting unit 38 obtains the multiple resin composition property prediction information by performing the property prediction using the mathematical model.

In step S210, the polymer design condition proposing unit 32 extracts an exhaustive search point in which the property prediction information on the polymer and the resin composition obtained in step S208 satisfies the required property information for the polymer and the resin composition as illustrated in FIG. 12, for example. The processing of step S210 is the process of extracting an exhaustive search point that has satisfied the required property information for the polymer and the resin composition as illustrated in FIG. 12 from among the exhaustive search points generated at random or at predetermined intervals within the range of the design condition.

In step S212, the control unit 30 performs control to cause the user terminal 12 to display the property prediction information and the design condition information for the polymer synthesized based on the exhaustive search point extracted in step S210 as information on the proposed polymer as illustrated in FIG. 15A and FIG. 15B, for example. For example, FIG. 15A indicates the property prediction information on the polymers synthesized based on the exhaustive search points extracted in step S210 as the information on the proposed polymers. Additionally, for example, FIG. 15B indicates the design requirement information on the polymers synthesized based on the exhaustive search points extracted in the step S210 as the information on the proposed polymers. The information on the proposed polymers illustrated in FIG. 15A and FIG. 15B is an example, and for example, the property prediction information on the proposed polymer illustrated in FIG. 15A and the design requirement information on the proposed polymer illustrated in FIG. 15B may be displayed side by side in one line.

Here, the control unit 30 may perform control to cause the user terminal 12 to display the information on the proposed polymer synthesized based on the exhaustive search points extracted in step S210, for example, as a calculation result screen 1100 as illustrated in FIG. 16. The calculation result screen 1100 of FIG. 16 is an example in which the information on the proposed polymers to be synthesized based on the exhaustive search points extracted in step S210 is graphically displayed.

In a field 1102, a slider for adjusting the required property is displayed on the calculation result screen 1100. By using the slider in the field 1102, the operator can plot calculation results obtained by adjusting the required property displayed in the field 1102.

In a field 1104, a graph of the molecular weights of multiple proposed polymers is displayed. In a field 1106, the property prediction information on multiple proposed polymers is displayed. Here, in the field 1106 of FIG. 16, the solvent resistance (for example, NMP resistance) and the heat resistance required when a resin composition is generated using the polymer are displayed as the property of the proposed polymer.

According to the process of FIG. 11, the design condition of the polymer when synthesizing the polymer from the monomers is searched and optimized, and then the property of the resin composition in which the polymerization initiator, the sensitizer, the adhesion agent, the plasticizer, and the like are mixed with the synthesized polymer according to predetermined evaluation conditions can be evaluated.

### <<Processing Example of Step S22>>

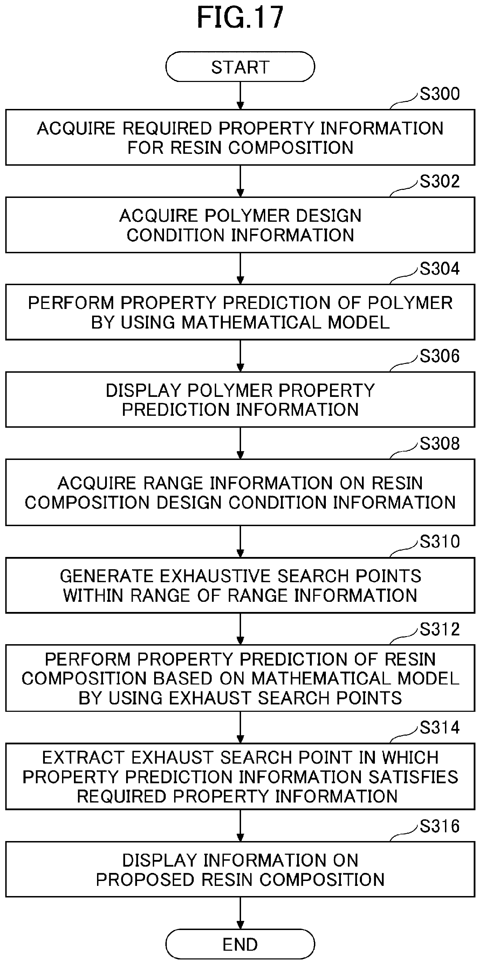

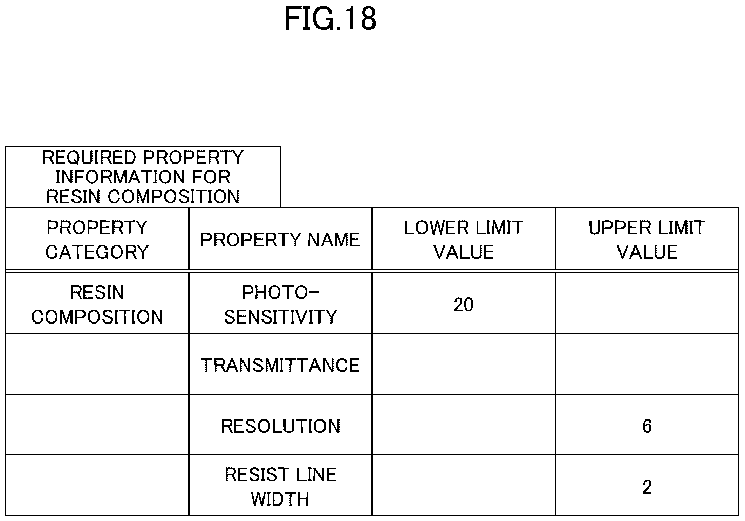

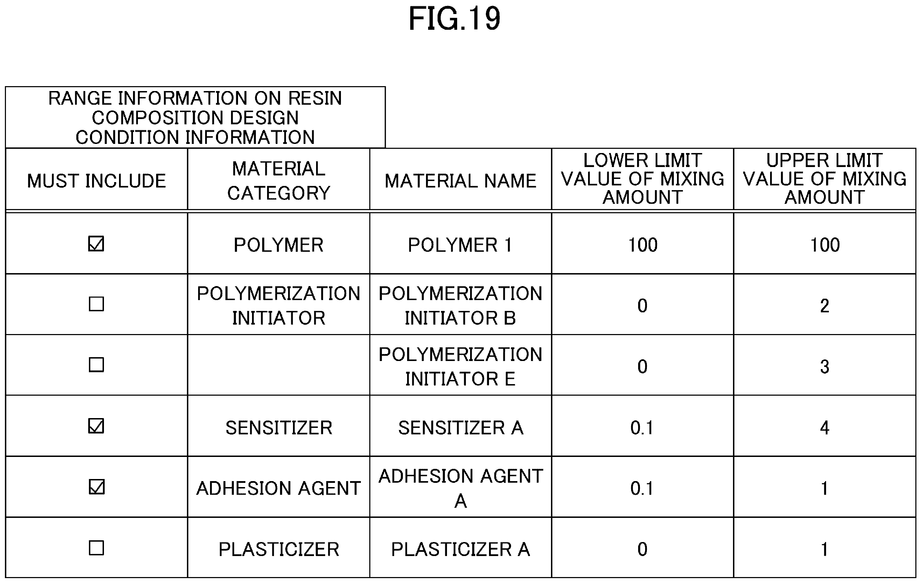

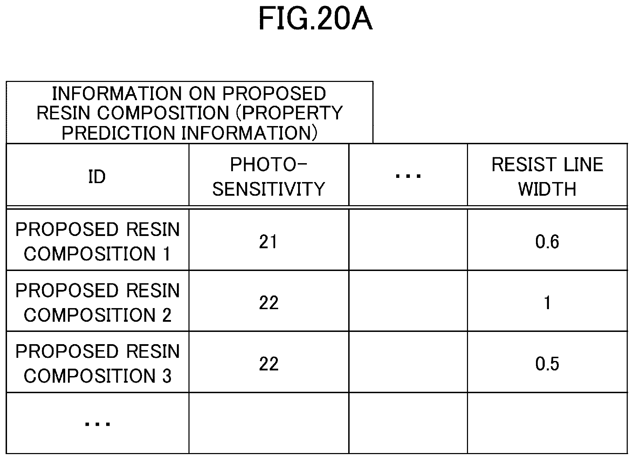

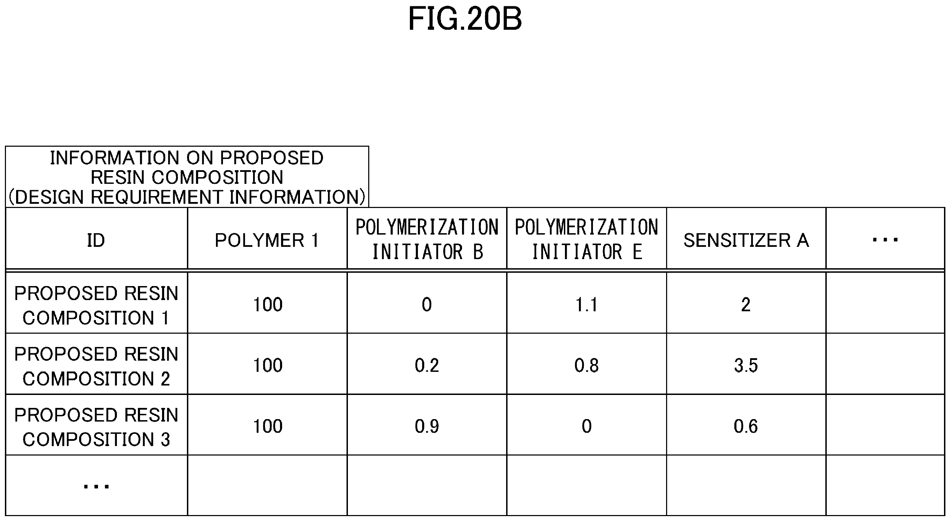

FIG. 17 is a flowchart of an example of the polymer design support process using the forward problem analysis and the resin composition design support process using the inverse problem analysis. FIG. 18 is a configuration diagram illustrating an example of the required property information for the resin composition. FIG. 19 is a configuration diagram illustrating an example of the range information on the resin composition design condition information. FIG. 20A and FIG. 20B are configuration diagrams illustrating an example of information on the proposed resin composition.

In step S300, the required property acquiring unit 26 of the design support device 10 acquires the required property information for the resin composition input by the operator at the user terminal 12, for example, as illustrated in FIG. 18. The required property information for the resin composition in FIG. 18 is an example in which the required property for the resin composition is represented by a lower limit value and an upper limit value.

In step S302, the design condition acquiring unit 28 of the design support device 10 acquires the polymer design condition information input by the operator at the user terminal 12, for example, as illustrated in FIG. 9A.

In step S304, the control unit 30 provides, to the polymer property predicting unit 36, the polymer design condition information acquired by the design condition acquiring unit 28 in step S302, and requests the property prediction of the polymer. The polymer property predicting unit 36 performs the property prediction based on the provided polymer design condition information by using the mathematical model. The polymer property predicting unit 36 obtains the polymer property prediction information, for example, as illustrated in FIG. 9B by the property prediction using the mathematical model.

In step S306, the control unit 30 performs control to cause the user terminal 12 to display the polymer property prediction information obtained in step S304, for example, as illustrated in FIG. 9B. The information that the user terminal 12 is caused to display in step S306 may include the polymer design condition information input by the operator in step S302, for example, illustrated in FIG. 9A.

In step S308, the design condition acquiring unit 28 acquires the range information on the resin composition design condition information input by the operator at the user terminal 12, for example, as illustrated in FIG. 19. The range information on the resin composition design condition information illustrated in FIG. 19 includes the designed polymer as a material. The range information on the resin composition design condition information in FIG. 19 includes, as information, the types of materials, and the lower limit value and the upper limit value of the mixing amount of each material.

Additionally, the range information on the resin composition design condition information in FIG. 19 is an example in which the operator can select a material necessary to be included in the resin composition to be generated. The item "must include" is checked for the material selected by the operator to be necessarily included in the resin composition to be generated. FIG. 19 is an example in which the operator selects "polymer 1", "sensitizer A", and "adhesion agent A" as the materials necessary to be included in the resin composition to be generated.

In step S310, the control unit 30 provides, to the resin composition design condition proposing unit 34, the range information on the resin composition design condition information acquired in step S308, and requests generation of exhaustive search points within the range of the range information on the resin composition design condition information. The resin composition design condition proposing unit 34 generates a predetermined number (for example, 1000) of exhaustive search points at random or at predetermined intervals within the range of the range information on the provided resin composition design condition information.

For example, in the range information on the resin composition design condition information in FIG. 19, the exhaustive search point is a combination of the mixing amounts of "polymer 1", "polymerization initiator B", "polymerization initiator E", "sensitizer A", "adhesion agent A", and "plasticizer A", which are the materials. The mixing amount of each material is selected within the range indicated by the items "lower limit value of mixing amount" and "upper limit value of mixing amount".

In step S312, the resin composition design condition proposing unit 34 performs the property prediction of the resin composition based on multiple exhaustive search points generated in step S310 by using the mathematical model, and obtains the property prediction information on multiple resin compositions based on multiple exhaustive search points.

In step S314, the resin composition design condition proposing unit 34 extracts an exhaustive search point in which the resin composition property prediction information obtained in step S312 satisfies the required property information for the resin composition, for example, as illustrated in FIG. 18. The processing of step S314 is the process of extracting the exhaustive search point that has satisfied the required property information for the resin composition as illustrated in FIG. 18 from among the exhaustive search points generated at random or at predetermined intervals within the range of the design conditions.

In step S316, the control unit 30 performs control to cause the user terminal 12 to display the property prediction information and the design condition information for the resin composition created based on the exhaustive search point extracted in step S314, for example, as the information on the proposed resin composition illustrated in FIG. 20A and FIG. 20B. For example, FIG. 20A indicates, as the information on the proposed resin composition, the property prediction information on the resin compositions synthesized based on the exhaustive search points extracted in step S314. Additionally, FIG. 20B indicates, as the information on the proposed resin composition, the design requirement information for the resin compositions synthesized based on the exhaustive search points extracted in step S314. The information on the proposed resin composition illustrated in FIG. 20A and FIG. 20B is an example, and the property prediction information on the proposed resin composition illustrated in FIG. 20A and the design requirement information on the proposed resin composition illustrated in FIG. 20B may be displayed side by side in one line, for example.

According to the process of FIG. 17, the property of the polymer synthesized from monomers under predetermined synthesis conditions is evaluated, and then a design condition of a resin composition in which a polymerization initiator, a sensitizer, an adhesion agent, a plasticizer, a solvent, and the like are mixed with the synthesized polymer can be searched for and optimized.

### <<Processing Example of Step S24>>

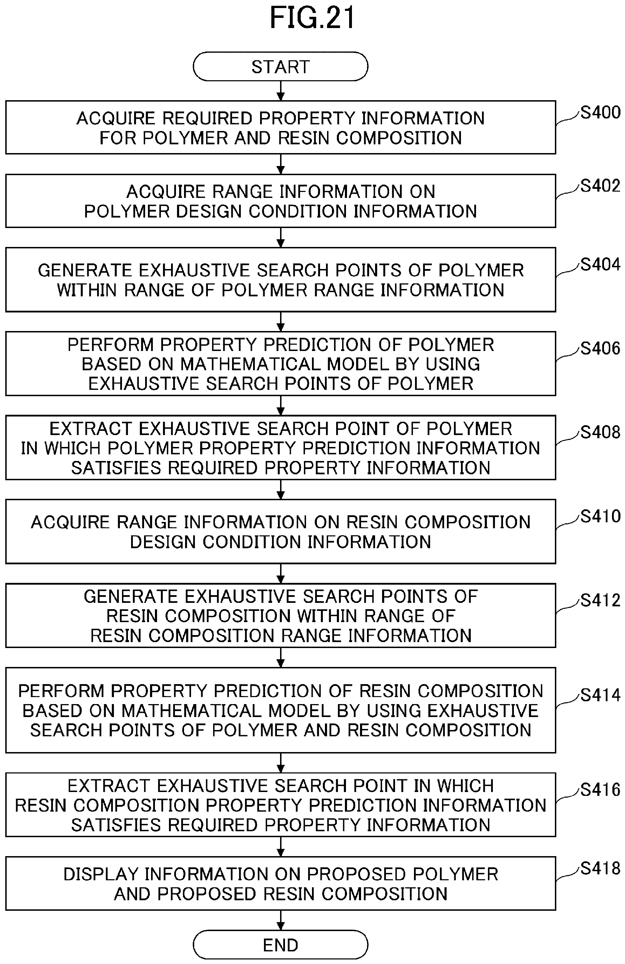

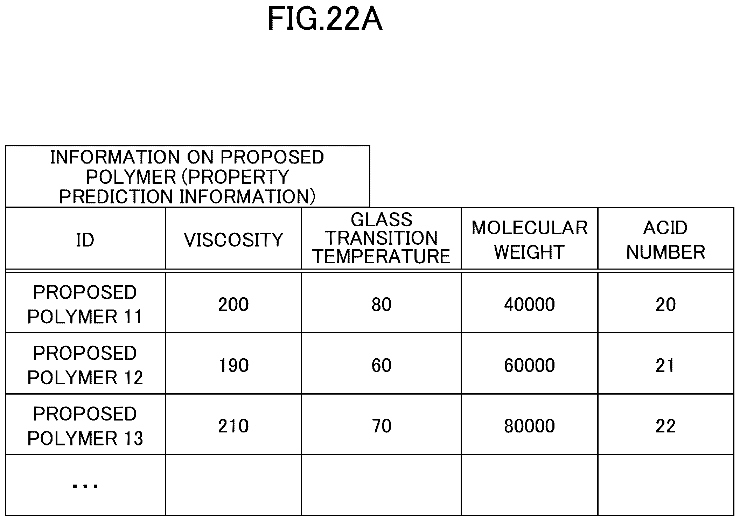

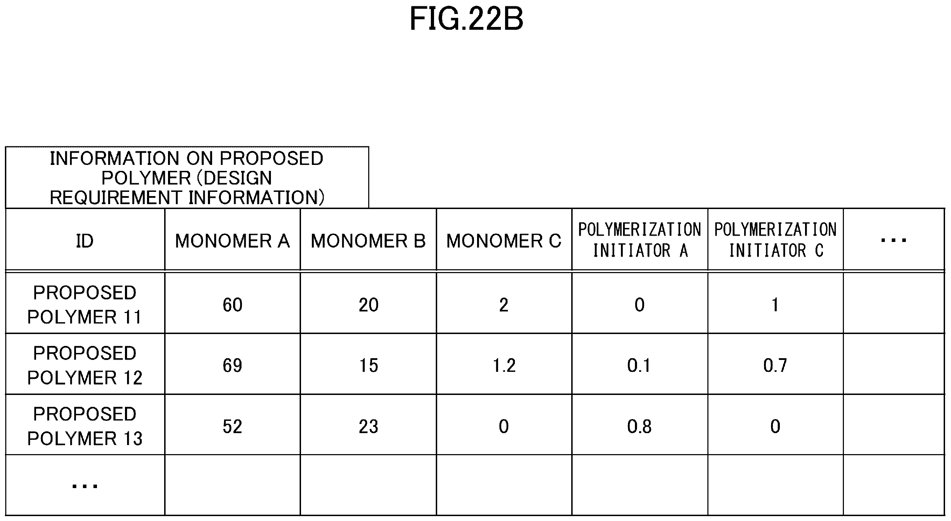

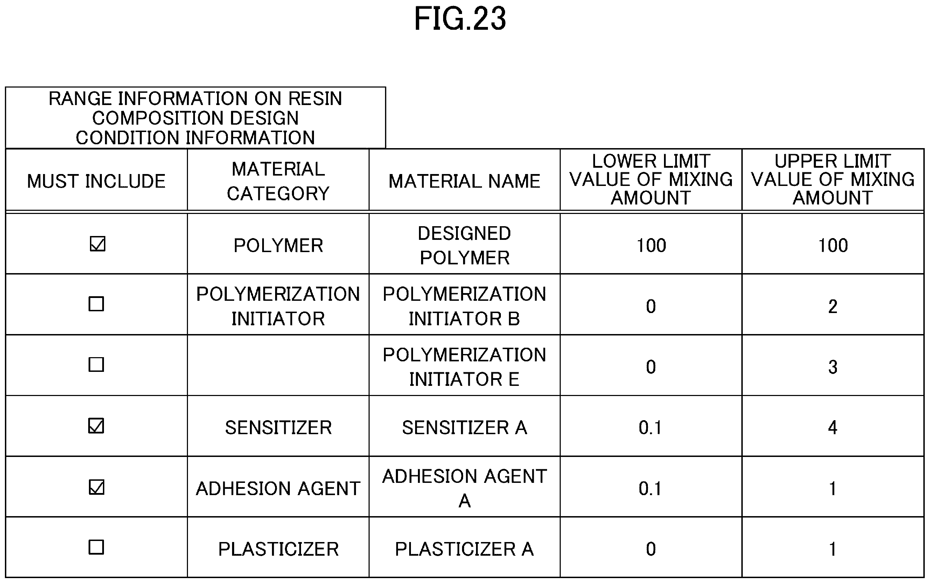

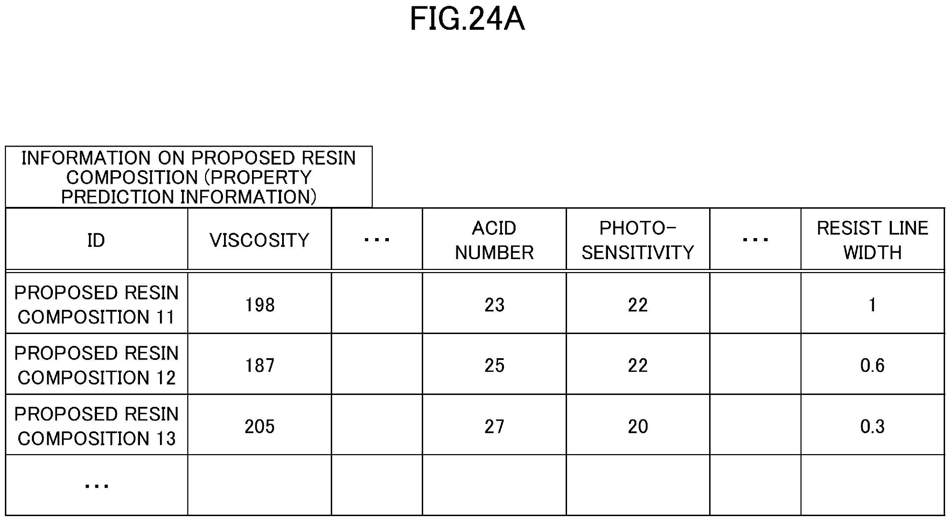

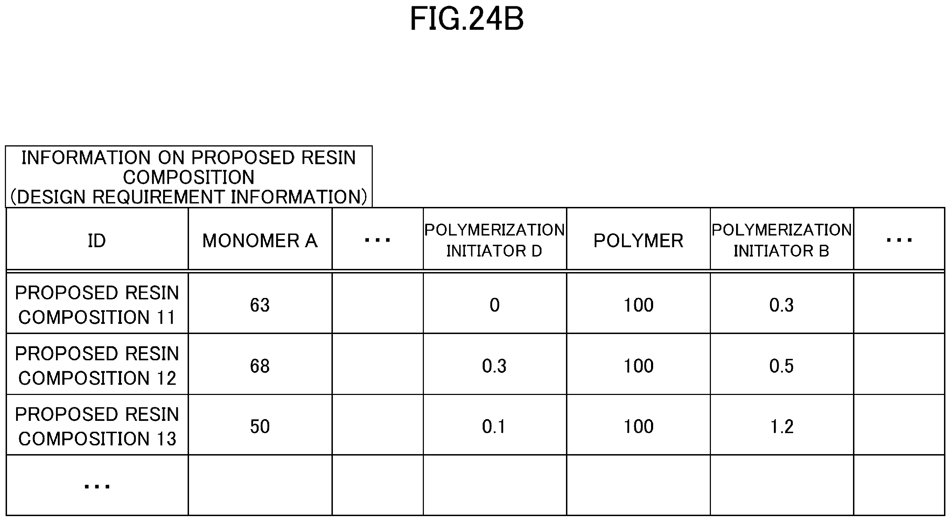

FIG. 21 is a flowchart of an example of the polymer design support process using the inverse problem analysis and the resin composition design support process using the inverse problem analysis. FIG. 22A and FIG. 22B are configuration diagrams illustrating an example of the information on the proposed polymer. FIG. 23 is a configuration diagram illustrating an example of the range information on the resin composition design condition information. FIG. 24A and FIG. 24B are configuration diagrams illustrating an example of the information on the proposed resin composition.

In step S400, the required property acquiring unit 26 of the design support device 10 acquires the required property information for the polymer and the resin composition input by the operator at the user terminal 12, for example, as illustrated in FIG. 12. In step S402, the design condition acquiring unit 28 acquires the range information on the polymer design condition information input by the operator at the user terminal 12, for example, as illustrated in FIG. 13.

In step S404, the control unit 30 provides, to the polymer design condition proposing unit 32, the range information on the polymer design condition information acquired in step S402, and requests generation of the exhaustive search points within the range of the range information on the polymer design condition information. The polymer design condition proposing unit 32 generates a predetermined number (for example, 1000) of exhaustive search points at random or at predetermined intervals within the range of the range information on the provided polymer design condition information.

In step S406, the polymer design condition proposing unit 32 performs the property prediction of the polymer based on the multiple exhaustive search points generated in step S404 by using the mathematical model, and obtains multiple polymer property prediction information based on the multiple exhaustive search points.

In step S408, the polymer design condition proposing unit 32 extracts an exhaustive search point in which the polymer property prediction information obtained in step S406 satisfies the required property information for the polymer, for example, as illustrated in FIG. 12. The processing of step S408 is the process of extracting the exhaustive search point that has satisfied the required property information for the polymer from among the exhaustive search points generated at random or with predetermined intervals within the range of the design condition of the polymer.

In step S410, the design condition acquiring unit 28 acquires the range information on the resin composition design condition information input by the operator at the user terminal 12, for example, as illustrated in FIG. 23. The range information on the resin composition design condition information illustrated in FIG. 23 includes the designed polymer (corresponding to the exhaustive search point extracted in step S408) as a material. The range information on the resin composition design condition information in FIG. 23 includes, as information, the types of materials, and the lower limit value and the upper limit value of the mixing amount of each material.

Additionally, the range information on the resin composition design condition information in FIG. 23 is an example in which the operator can select a material necessary to be included in the resin composition to be generated. The item "must include" is checked for the material selected by the operator to be necessarily included in the resin composition to be generated. FIG. 23 indicates an example in which the operator selects the "designed polymer", the "sensitizer A", and the "adhesion agent A" as the materials necessary to be included in the resin composition to be generated.

In step S412, the control unit 30 provides, to the resin composition design condition proposing unit 34, the range information on the resin composition design condition information acquired in step S410, and requests generation of the exhaustive search points within the range of the range information on the resin composition design condition information. The resin composition design condition proposing unit 34 generates a predetermined number (for example, 1000) of exhaustive search points of the resin composition at random or at predetermined intervals within the range of the range information on the provided resin composition design condition information.

For example, in the range information on the resin composition design condition information in FIG. 23, the exhaustive search point of the resin composition is a combination of the mixing amounts of the "designed polymer", the "polymerization initiator B", the "polymerization initiator E", the "sensitizer A", the "adhesion agent A", and the "plasticizer A", which are the materials. The mixing amount of each material is selected from the range indicated by the items "lower limit value of mixing amount" and "upper limit value of mixing amount".

In step S414, the resin composition design condition proposing unit 34 performs the property prediction of the resin composition based on the multiple exhaustive search points of the polymer extracted in step S408 and the multiple exhaustive search points of the resin composition generated in step S412 by using the mathematical model. The resin composition design condition proposing unit 34 obtains multiple resin composition property prediction information based on the multiple exhaustive search points of the polymer and the resin composition.

In step S416, the resin composition design condition proposing unit 34 extracts exhaustive search points of the polymer and the resin composition in which the resin composition property prediction information obtained in step S414 satisfies the required property information for the resin composition, for example, as illustrated in FIG. 12.

In step S418, the control unit 30 performs control to cause the user terminal 12 to display the property prediction information and the design condition information for the polymer synthesized based on the exhaustive search point of the polymer extracted in step S416 as the information on the proposed polymer, for example, as illustrated in FIG. 22A and FIG. 22B. FIG. 22A illustrates, as the information on the proposed polymer, the property prediction information on the polymers synthesized based on the exhaustive search points extracted in step S416, for example. Additionally, FIG. 22B illustrates, as the information on the proposed polymer, the design requirement information on the polymers synthesized based on the exhaustive search points extracted in step S416. Here, the information on the proposed polymer illustrated in FIG. 22A and FIG. 22B is an example, and the property prediction information on the proposed polymer illustrated in FI. 22A and the design requirement information on the proposed polymer illustrated in FIG. 22B may be displayed side by side in one line, for example.

Additionally, in step S418, the control unit 30 performs control to cause the user terminal 12 to display the property prediction information and the design condition information for the resin-composition generated based on the exhaustive search point extracted in step S416, for example, as the information on the proposed resin composition as illustrated in FIG. 24A and FIG. 24B. FIG. 24A illustrates, as the information on the proposed resin composition, the property prediction information on the resin compositions synthesized based on the exhaustive search points extracted in step S416, for example. Additionally, FIG. 24B illustrates, as the information on the proposed resin composition, the design requirement information on the resin compositions synthesized based on the exhaustive search points extracted in the step S416. The information on the proposed resin composition illustrated in FIG. 24A and FIG. 24B is an example, and the property prediction information on the proposed resin composition illustrated in FIG. 24A and the design requirement information on the proposed resin composition illustrated in FIG. 24B may be displayed side by side in one line, for example.

According to the process of FIG. 21, by narrowing down the exhaustive search points of the polymer that satisfy the required property of the polymer design in advance, it becomes unnecessary to generate the exhaustive search points of the resin composition containing the polymer that do not satisfy the required property of the polymer design or to perform the property prediction, and the design condition of the resin composition can be efficiently searched.

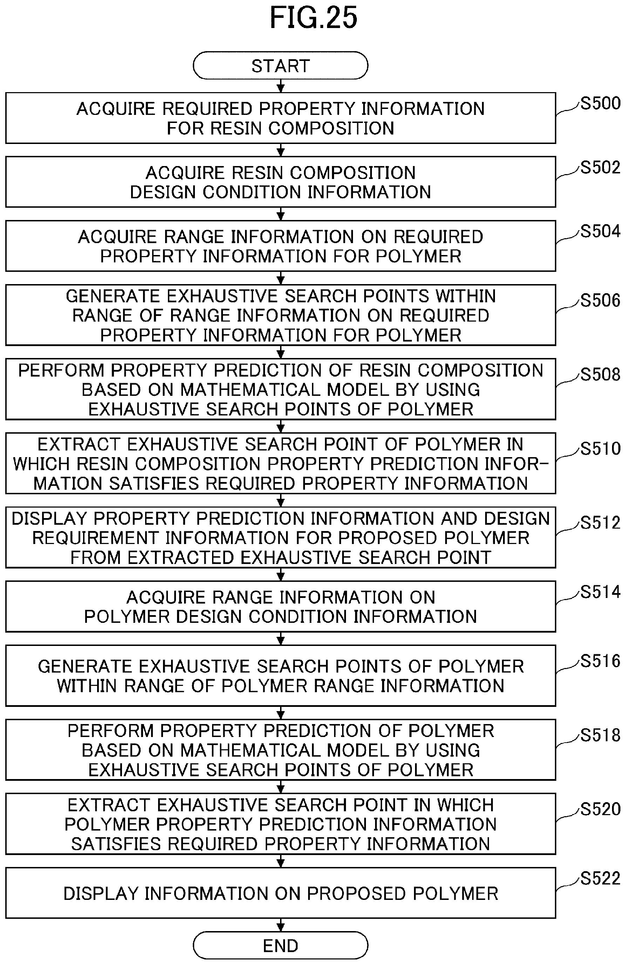

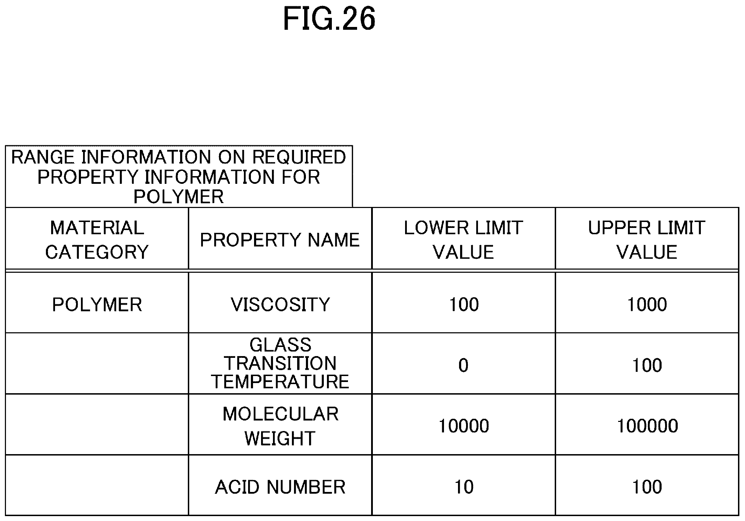

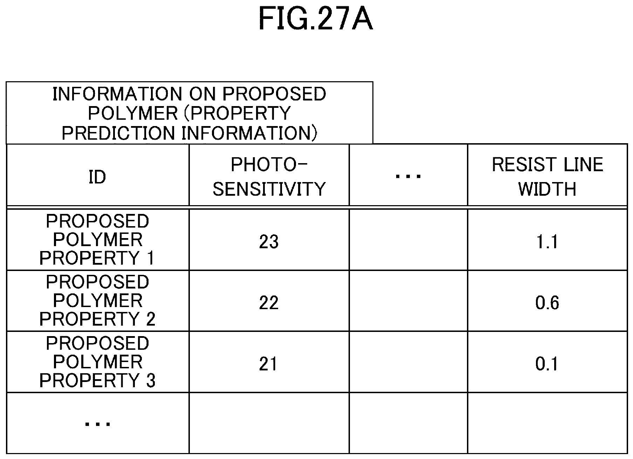

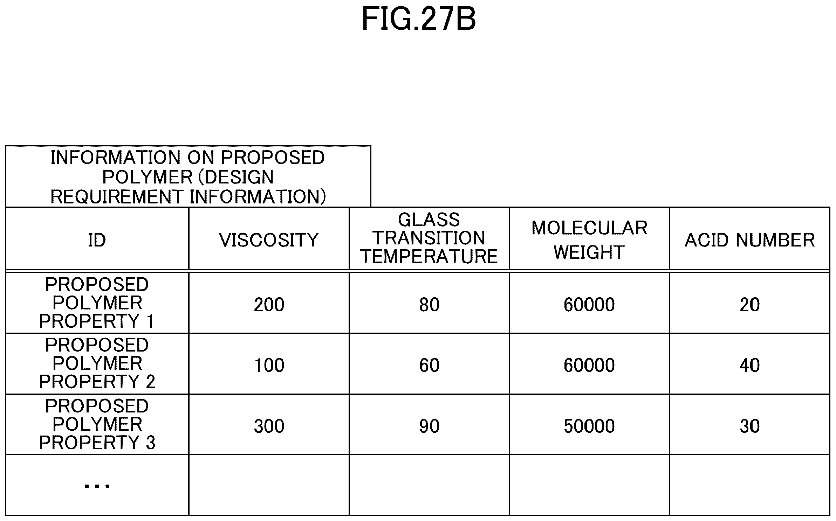

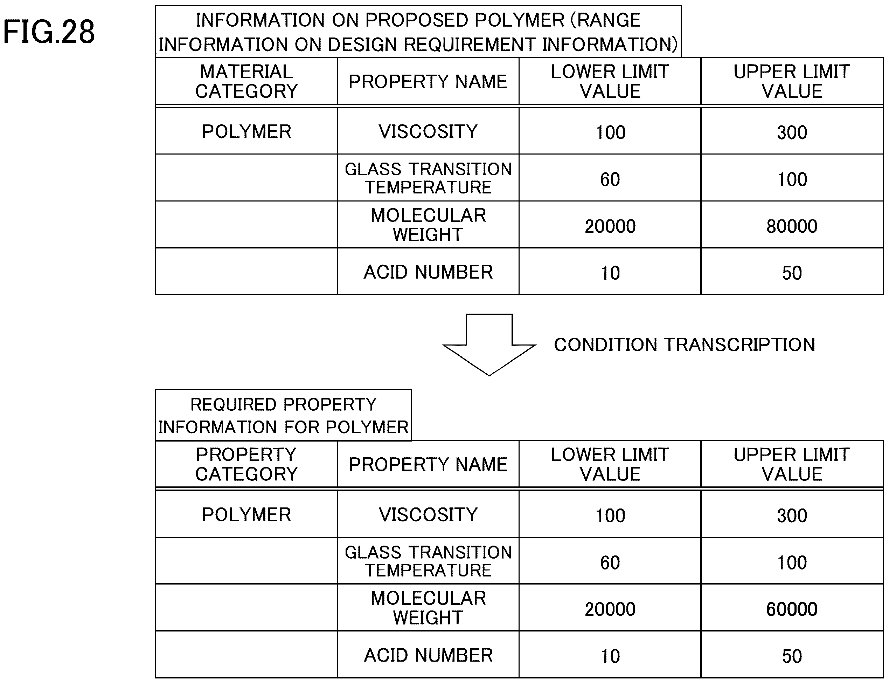

FIG. 25 is a flowchart of an example of the polymer design support process using the inverse problem analysis and the resin composition design support process using the inverse problem analysis. FIG. 26 is a configuration diagram illustrating an example of the range information on the polymer property prediction information. FIG. 27A and FIG. 27B are configuration diagrams illustrating an example of the information on the proposed polymer. FIG. 28 is a configuration diagram illustrating an example of the required property information for the polymer transcribed from the information on the proposed polymer.

In step S500, the required property acquiring unit 26 of the design support device 10 acquires the required property information for the resin composition input by the operator at the user terminal 12, for example, as illustrated in FIG. 18. In step S502, the design condition acquiring unit 28 acquires the resin composition design condition information input by the operator at the user terminal 12, for example, as illustrated in FIG. 14.

In step S504, the required property acquiring unit 26 acquires the range information on the required property information for the polymer input by the operator at the user terminal 12, for example, as illustrated in FIG. 26. In the range information on the required property information for the polymer in FIG. 26, the required property for the polymer is represented by a lower limit value and an upper limit value for each required property.

In step S506, the control unit 30 provides, to the polymer design condition proposing unit 32, the range information on the required property information for the polymer acquired in step S504, and requests generation of the exhaustive search points within the range of the range information on the required property information for the polymer. The polymer design condition proposing unit 32 generates a predetermined number (for example, 1000) of exhaustive search points at random or at predetermined intervals within the range of the range information on the provided required property information for the polymer.

In step S508, the control unit 30 requests the resin composition design condition proposing unit 34 to perform the property prediction of the resin composition based on the exhaustive search points of the polymer generated in step S506. The resin composition design condition proposing unit 34 performs the property prediction of the resin composition based on the exhaustive search points of the polymers generated in step S506 by using the mathematical model, and obtains the multiple resin composition property prediction information based on the multiple exhaustive search points.

In step S510, the polymer design condition proposing unit 32 extracts, from among the multiple exhaustive search points generated in step S506, an exhaustive search point in which the resin composition property prediction information obtained in step S508 satisfies the required property information for the resin composition, for example, as illustrated in FIG. 18. The processing of step S510 is the process of extracting the exhaustive search point that has satisfied the required property information for the resin composition as illustrated in FIG. 18 from among the exhaustive search points generated at random or at predetermined intervals in the range of the required property of the polymer, for example.

In step S512, the control unit 30 performs control to cause the user terminal 12 to display the property prediction information and the design requirement information on the polymer synthesized based on the exhaustive search point extracted in step S510 as the information on the proposed polymer, for example, as illustrated in FIG. 27A and FIG. 27B. FIG. 27A illustrates, as the information on the proposed polymer, the property prediction information on the polymers synthesized based on the exhaustive search points extracted in step S510, for example. Additionally, FIG. 27B illustrates, as the information on the proposed polymer, the design requirement information on the polymers synthesized based on the exhaustive search points extracted in step S510, for example. The information on the proposed polymer illustrated in FIG. 27A and FIG. 27B is an example, and the property prediction information on the proposed polymer illustrated in FIG. 27A and the design requirement information on the proposed polymer illustrated in FIG. 27B may be displayed side by side in one line, for example.

In step S512, the control unit 30 performs control to cause the user terminal 12 to display the range information on the polymer design requirement information acquired from the proposed polymer design requirement information illustrated in the FIG. 27B as the information on the proposed polymer as illustrated in the upper part of FIG. 28, for example. In the upper part of FIG. 28, for example, the range information on the polymer design requirement information is proposed to the operator as the information on the proposed polymer. Here, as illustrated in FIG. 28, the operator can transcribe the information on the proposed polymer in the upper part as the required property information for the polymer illustrated in the lower part, and can also correct the numerical value after the transcription.

In step S514, the design condition acquiring unit 28 acquires the range information on the polymer design condition information input by the operator at the user terminal 12, for example, in FIG. 13.

In step S516, the control unit 30 provides, to the polymer design condition proposing unit 32, the range information on the polymer design condition information acquired in step S514, and requests generation of the exhaustive search points within the range of the range information on the polymer design condition information. The polymer design condition proposing unit 32 generates a predetermined number (for example, 1000) of exhaustive search points at random or at predetermined intervals within the range of the range information on the provided polymer design condition information.

In step S518, the polymer design condition proposing unit 32 performs the property prediction of the polymer based on the multiple exhaustive search points generated in step S516 by using the mathematical model, and obtains multiple polymer property prediction information based on the multiple exhaustive search points.

In step S520, the polymer design condition proposing unit 32 extracts an exhaustive search point in which the polymer property prediction information obtained in step S518 satisfies the required property information for the polymer as illustrated in the lower part of FIG. 28, for example. The processing of step S520 is the process of extracting the exhaustive search point that has satisfied the required property information for the polymer from among the exhaustive search points generated at random or with predetermined intervals within the range of the design condition of the polymer.

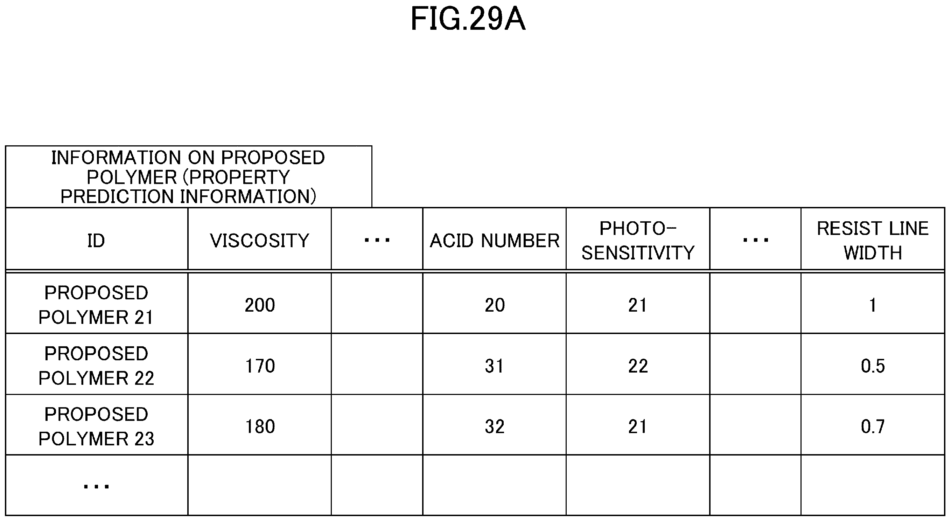

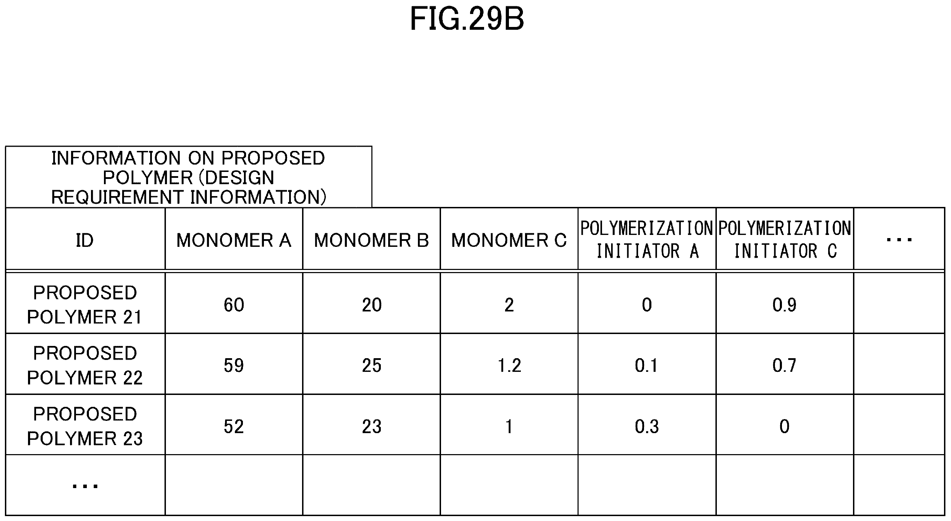

In step S522, the control unit 30 performs control to cause the user terminal 12 to display the property prediction information and the design condition information for the polymer synthesized based on the exhaustive search point of the polymer extracted in step S520 as the information on the proposed polymer as illustrated in FIG. 29A and FIG. 29B, for example. FIG. 29A illustrates, as the information on the proposed polymer, the property prediction information on the polymers synthesized based on the exhaustive search points extracted in step S520, for example. Additionally, FIG. 29B illustrates, as the information on the proposed polymer, the design requirement information on the polymers synthesized based on the exhaustive search points extracted in the step S520. Here, the information on the proposed polymer illustrated in FIG. 29A and FIG. 29B is an example, and the property prediction information on the proposed polymer illustrated in FIG. 29A and the design requirement information on the proposed polymer illustrated in FIG. 29B may be displayed side by side in one line, for example.

According to the process of FIG. 25, the property of the polymer that satisfies the required property for the resin composition can be searched, and further, the design condition of the polymer that satisfies the property of the polymer can be searched and optimized.

When designing the resin composition, the properties of the resin composition may be controlled by adjusting the properties of the polymer such as the viscosity, the glass transition point, the molecular weight, the acid value, and the like. Therefore, it is necessary to search for the properties of the polymer that satisfy the required properties for the resin composition, and then search for and optimize the compositions of the monomer, the polymerization initiator, and the like that satisfy the property of the polymer, and the design support device 10 that is suitable is required.

Additionally, an operator who performs the mix of the resin composition may perform development while combining the properties of the polymer and the properties of the resin composition in his/her head. Therefore, it is very convenient that the properties of the polymer that satisfy the required properties of the resin composition are proposed. Additionally, an operator who performs the synthesis of the polymer may select a monomer to be used and performs development while imagining properties of a target polymer in his/her head. Therefore, it is very convenient that the properties of the polymer that satisfy the required properties for the resin composition are proposed.

### <<Registration and Use of Design Condition Information and Property Prediction Information»

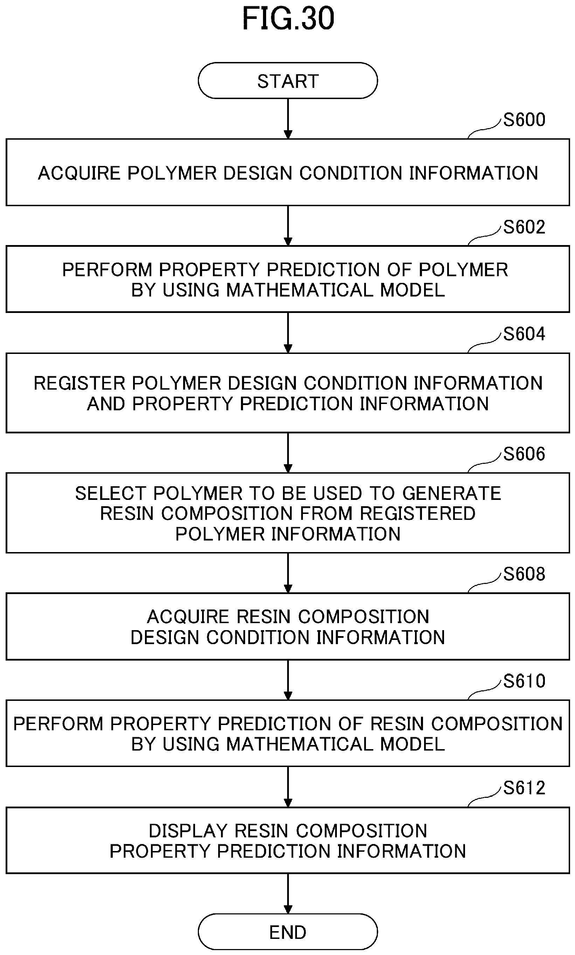

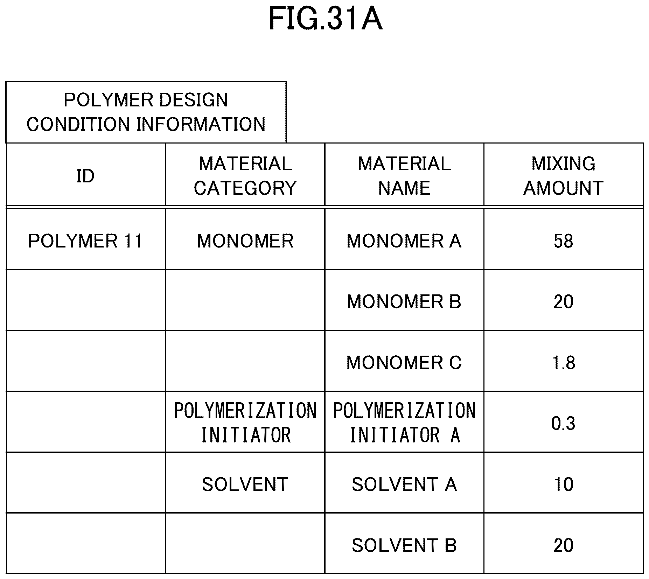

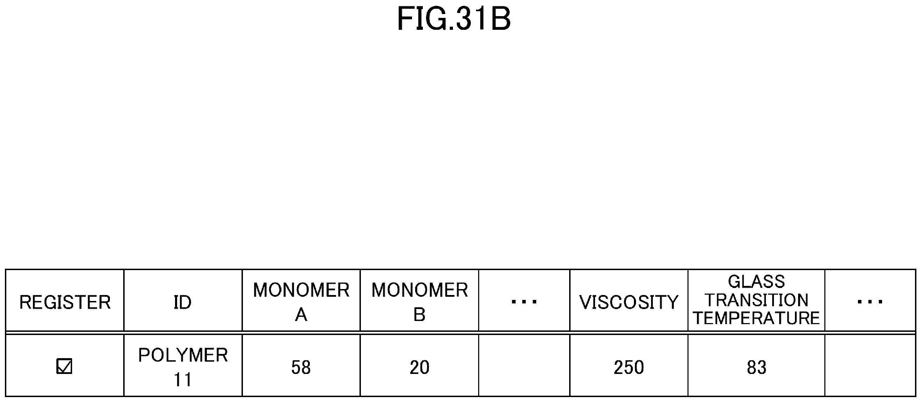

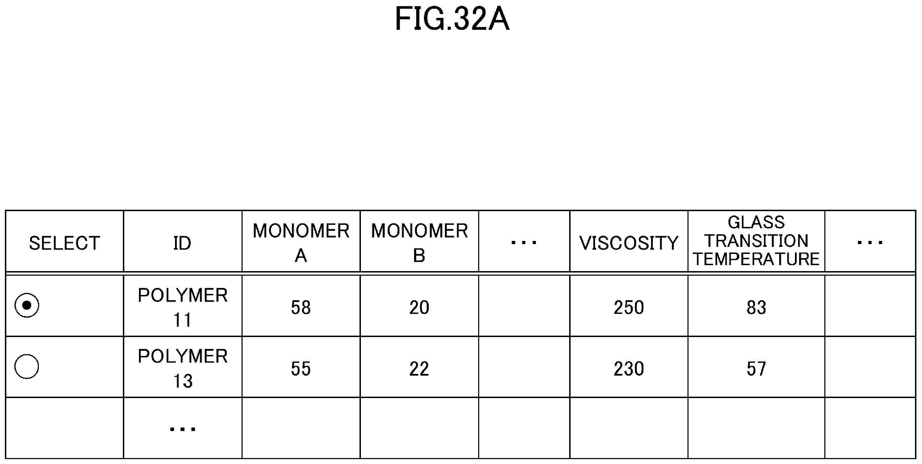

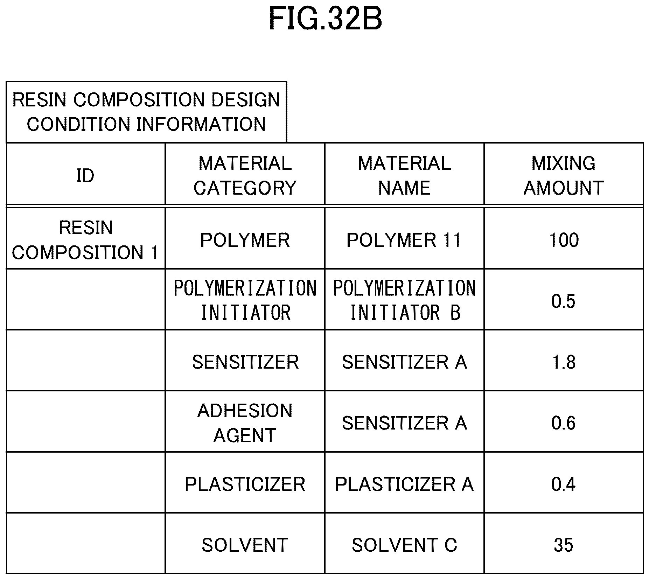

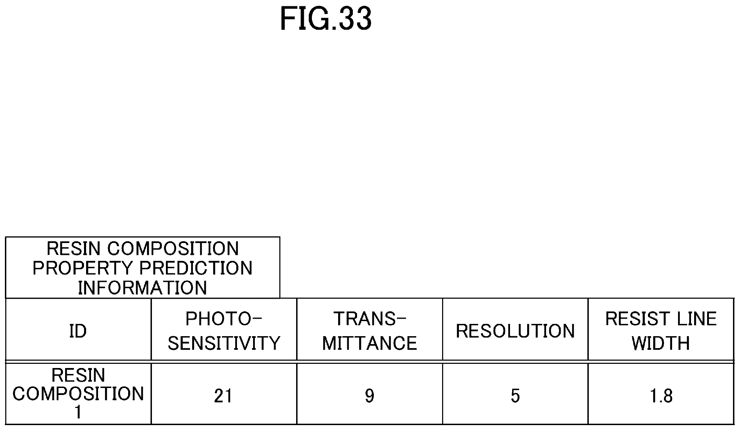

FIG. 30 is a flowchart of an example of a registration and use process of the design condition information and the property prediction information. FIG. 31A is a configuration diagram illustrating an example of the design condition information for the polymer. FIG. 31B is a configuration diagram illustrating an example of the polymer property prediction information. FIG. 32A and FIG. 32B are configuration diagrams illustrating an example of a registration and use process of the design condition information and the property prediction information on the polymer. FIG. 33 is a configuration diagram illustrating an example of the resin composition property prediction information.

In step S600, the design condition acquiring unit 28 of the design support device 10 acquires the polymer design condition information input by the operator at the user terminal 12, for example, as illustrated in FIG. 31A. The polymer design condition information includes the types and mixing amounts of the materials as information.

In step S602, the control unit 30 provides, to the polymer property predicting unit 36, the polymer design condition information acquired by the design condition acquiring unit 28 in step S600, and requests the property prediction of the polymer. The polymer property predicting unit 36 performs the property prediction based on the provided polymer design condition information by using the mathematical model. The polymer property predicting unit 36 obtains the polymer property prediction information by the property prediction using the mathematical model.

In step S604, the control unit 30 performs control to cause the user terminal 12 to display the polymer property prediction information obtained in step S602. The control unit 30 causes the user terminal 12 to display the design condition information and the property prediction information on the polymer illustrated in FIG. 31B, for example, so that the operator can select the polymer to be registered. In the example of FIG. 31B, the design condition information and the property prediction information on the polymer for which the item "register" is checked are associated with each other and registered in the design support device 10.

In step S606, the control unit 30 can select a polymer to be used for generating a resin composition from the information on the registered polymers, for example, as illustrated in FIG. 32A. The operator can select the polymer to be used as the resin composition design condition information, for example, as illustrated in FIG. 32B from the information on the polymers registered by the radio button of the item "register" in FIG. 32A.

In step S608, the design condition acquiring unit 28 of the design support device 10 acquires the resin composition design condition information input by the operator at the user terminal 12, for example, as illustrated in FIG. 32B. The resin composition design condition information illustrated in FIG. 32B includes the registered polymer selected in the step S606 as a material.

In step S610, the control unit 30 provides, to the resin composition property predicting unit 38, the resin composition design condition information acquired by the design condition acquiring unit 28 in step S608, and requests the property prediction of the resin composition. The resin composition property predicting unit 38 performs the property prediction based on the provided resin composition design condition information by using the mathematical model. The resin composition property predicting unit 38 obtains the resin composition property prediction information, for example, illustrated in FIG. 33 by the property prediction using the mathematical model. In step S612, the control unit 30 performs control to cause the user terminal 12 to display the resin composition property prediction information, for example, as illustrated in FIG. 33.

According to the process of FIG. 30, the design condition information and the property prediction information on the polymer whose design has been completed are registered, and can be selected and used when the resin composition is designed. For example, in the development of the resin composition, the evaluation of the property of the resin composition is frequently performed by using a polymer whose design has been completed multiple times and variously changing the types and mixing amounts of the polymerization initiator, the sensitizer, the adhesion agent, the plasticizer, and the like.

Therefore, it is very convenient that the design condition information and the property prediction information on a polymer whose design has been completed are registered, and can be selected and used when a resin composition is designed.

Here, the registration and use of the design condition information and the property prediction information on the polymer in the combination of the design of the polymer by the inverse problem analysis and the property prediction of the resin composition by the forward problem analysis have been described above. However, the same applies to the combination of the design of the polymer by the forward problem analysis and the property prediction of the resin composition by the inverse problem analysis, the combination of the design of the polymer by the forward problem analysis and the property prediction of the resin composition by the forward problem analysis, or the combination of the design of the polymer by the inverse problem analysis and the property prediction of the resin composition by the inverse problem analysis.

### [Other Embodiments]

With respect to the polymer designed by the design support device 10 according to the present embodiment and the resin composition containing the polymer, by supplying design condition information for the polymer and a compound such as the resin composition containing the polymer to a manufacturing device that generates the polymer and the compound such as the resin composition containing the polymer, the polymer and the compound such as the resin composition containing the polymer may be generated by the manufacturing device.

As described above, according to the information processing system of the present embodiment, a design support device, a design support method, and a program suitable for designing a first compound such as a polymer and designing a second compound such as a resin composition containing the first compound such as a polymer can be provided.

While the present embodiments have been described above, it will be understood that various changes in form and detail may be made without departing from the spirit and scope of the appended claims.

Although the present invention has been described based on the embodiments, the present invention is not limited to the above-described embodiments, and various modifications can be made within the scope described in the claims. This application claims priority to Basic Application No. 2021-206288 filed on December 20, 2021 with the Japan Patent Office, the entire contents of which are incorporated herein by reference.

### Description of Reference Symbols

- 10: design support device

- 12: user terminal
- 18: communication network
- 24: design mode acquiring unit
- 26: required property acquiring unit
- 28: design condition acquiring unit
- 30: control unit
- 32: polymer design condition proposing unit
- 34: resin composition design condition proposing unit
- 36: polymer property predicting unit
- 38: resin composition property predicting unit
- 40: storage unit
- 502: display device

## Claims

1. A design support device that supports design of a first compound and design of a second compound containing the first compound, the design support device comprising:
a first design condition proposing unit configured to propose a candidate for design condition information for the first compound that satisfies required property information for the first compound that is input; and
a first property predicting unit configured to perform property prediction of the second compound based on design condition information for the second compound that is input.

2. The design support device according to claim 1, further comprising:
a second design condition proposing unit configured to propose a candidate for design condition information for the second compound that satisfies required property information for the second compound that is input;
a second property predicting unit configured to perform property prediction of the first compound based on design condition information for the first compound that is input; and
a control unit configured to support design of the first compound by using the first design condition proposing unit or the second property predicting unit, and support design of the second compound by using the second design condition proposing unit or the first property predicting unit.

3. A design support device that supports design of a first compound and design of a second compound containing the first compound, the design support device comprising:
a second property predicting unit configured to perform property prediction of the first compound based on design condition information for the first compound that is input; and
a second design condition proposing unit configured to propose a candidate for design condition information for the second compound that satisfies required property information for the second compound that is input.

4. The design support device according to claim 3, further comprising:
a first design condition proposing unit configured to propose a candidate for design condition information for the first compound that satisfies required property information for the first compound that is input;
a first property predicting unit configured to perform property prediction of the second compound based on design condition information for the second compound that is input; and
a control unit configured to support design of the first compound by using the first design condition proposing unit or the second property predicting unit, and support design of the second compound by using the second design condition proposing unit or the first property predicting unit.

5. The design support device according to claim 2 or 4, wherein the control unit is configured to perform the property prediction of the first compound based on the design condition information for the first compound by using the second property predicting unit, and perform the property prediction of the second compound based on the design condition information for the second compound containing the first compound of which the property prediction is performed, by using the first property predicting unit.

6. The design support device according to claim 2 or 4, wherein the control unit is configured to propose the candidate for the design condition information for the first compound that satisfies the required property information for the first compound by using the first design condition proposing unit, and perform the property prediction of the second compound based on the design condition information for the second compound containing the first compound of the candidate for the design condition information by using the first property predicting unit.

7. The design support device according to claim 2 or 4, wherein the control unit is configured to perform the property prediction of the first compound based on the design condition information for the first compound by using the second property predicting unit, and propose the candidate for the design condition information for the second compound that satisfies the required property information for the second compound containing the first compound of which the property prediction is performed, by using the second design condition proposing unit.

8. The design support device according to claim 2 or 4, wherein the control unit is configured to propose the candidate for the design condition information for the first compound that satisfies the required property information for the first compound by using the first design condition proposing unit, and propose the candidate for the design condition information for the second compound that satisfies the required property information for the second compound containing the first compound of the candidate for the design condition information by using the second design condition proposing unit.

9. The design support device according to claim 8, wherein the second design condition proposing unit is configured to propose a candidate for a property of the first compound that satisfies the required property information for the second compound.

10. The design support device according to any one of claims 2, 4, 5, and 7, the design support device further comprising:
a storage unit configured to store a result of the property prediction of the first compound performed by the second property predicting unit based on the design condition information for the first compound,
wherein the first property predicting unit is configured to use the design condition information for the first compound and the result of the property prediction of the first compound that are read from the storage unit for input of the design condition information for the second compound and for the property prediction of the second compound.

11. The design support device according to any one of claims 2 to 10, wherein the first design condition proposing unit and the second design condition proposing unit are configured to receive the required property information and range information on the design condition information as inputs, and propose the candidate for the design condition information for the first compound or the second compound that satisfies the required property information for the first compound or the second compound within a range of the range information that is input.

12. The design support device according to claim 11, wherein the first design condition proposing unit and the second design condition proposing unit are configured to generate an exhaustive search point within the range of the range information that is input and propose the candidate for the design condition information for the first compound or the second compound that satisfies the required property information for the first compound or the second compound based on a result of the property prediction of the first compound or the second compound performed using the exhaustive search point.

13. The design support device according to any one of claims 1 to 12, wherein the first compound is a polymer polymerized from monomers, and the second compound is a resin composition containing the polymer.

14. A design support method of a computer supporting design of a first compound and design of a second compound containing the first compound, the design support method comprising:
a design condition proposing step of proposing a candidate for design condition information for the first compound that satisfies required property information for the first compound that is input; and
a property predicting step of performing property prediction of the second compound based on design condition information for the second compound that is input.

15. A program for causing a computer configured to support design of a first compound and design of a second compound containing the first compound to execute:
a design condition proposing procedure of proposing a candidate for design condition information for the first compound that satisfies required property information for the first compound that is input; and
a property predicting procedure of performing property prediction of the second compound based on design condition information for the second compound that is input.
